# EUROPEAN PATENT SPECIFICATION

(11) **EP 4 094 679 B1**
(45) Date of publication and mention of the grant of the patent: **23.09.2026**
(21) Application number: 22174101.0
(22) Date of filing: 18.05.2022
(51) Int. Cl.: A61B 5/00, A61B 5/11, A61B 5/08, A61B 5/024, A61B 5/05

(54) **MILLIMETER WAVE (MMWAVE) MAPPING SYSTEMS FOR GENERATING ONE OR MORE POINT CLOUDS AND DETERMINING ONE OR MORE VITAL SIGNS FOR DEFINING A HUMAN PSYCHOLOGICAL STATE**
MILLIMETERWELLEN-(MMWAVE)-ABBILDUNGSSYSTEME ZUM ERZEUGEN EINER ODER MEHRERER PUNKTWOLKEN UND ZUM BESTIMMEN EINES ODER MEHRERER VITALZEICHEN ZUM DEFINIEREN EINES MENSCHLICHEN PSYCHOLOGISCHEN ZUSTANDS
SYSTÈMES DE CARTOGRAPHIE À ONDES MILLIMÉTRIQUES (MMWAVE) POUR GÉNÉRER UN OU PLUSIEURS NUAGES DE POINTS ET DÉTERMINER UN OU PLUSIEURS SIGNES VITAUX POUR DÉFINIR UN ÉTAT PSYCHOLOGIQUE HUMAIN

(30) Priority: 26.05.2021 US 202163193128 P
(43) Date of publication of application: 30.11.2022
(73) Proprietor: The Procter & Gamble Company, Cincinnati, OH 45202 (US)
(72) Inventor: KHAN, Nasir Saeed, 61476 Kronberg (DE); HITTMEYER, Claus, 65824 Schwalbach (DE); SHERMAN, Faiz Feisal, Cincinnati, 45202 (US)
(74) Representative: P&G Patent Germany

(56) References cited:
- WO-A1-2021/067860
- CN-A- 109 276 384
- US-A1- 2016 310 697
- US-A1- 2019 254 544
- US-A1- 2020 178 892
- US-A1- 2020 300 972
- US-B2- 10 576 328
- YANG ZHICHENG ZCYANG@UCDAVIS EDU ET AL: "Vital Sign and Sleep Monitoring Using Millimeter Wave", ACM TRANSACTIONS ON SENSOR NETWORKS, ACM, 2 PENN PLAZA, SUITE 701 NEW YORK NY 10121-0701 USA, vol. 13, no. 2, 24 April 2017 (2017-04-24), pages 1 - 32, XP058691396, ISSN: 1550-4859, DOI: 10.1145/3051124
- SINGH ANURADHA ET AL: "Multi-Resident Non-Contact Vital Sign Monitoring Using Radar: A Review", IEEE SENSORS JOURNAL, IEEE, USA, vol. 21, no. 4, 5 November 2020 (2020-11-05), pages 4061 - 4084, XP011833264, ISSN: 1530-437X, [retrieved on 20210118], DOI: 10.1109/JSEN.2020.3036039

## Description

### FIELD

The present disclosure generally relates to millimeter wave (mmWave) mapping systems and, more particularly, to mmWave mapping systems for generating one or more point clouds and determining one or more vital signs for defining a human psychological state.

### BACKGROUND

Conventional monitoring technology, such as sleep monitoring technology, can be invasive to a user's privacy or personally identifiable information ("PII"). Typically such PII comprises image or video technology, where a user is imaged in his or her private residence. While such monitory technologies can be useful, such as viewing a sleeping child in a different room of a residence, the technology can be at times overly invasive and create privacy and/or data risks of a person's Pll in today's increasingly digitally connected world.

For example, PII, such as pictures, video, and/or sound of a user, as captured by monitoring device, may be susceptible to security breaches, such as cyber-attacks via computer networks to which the monitoring technology is connected. This can create a security risk to the user of inadvertently losing control of PII. In addition, many countries and/or regulatory bodies increasingly require the protection of PII and other private data of a person, which existing monitoring technology no longer complies with. For example, the General Data Protection Regulation (GDPR) of the European Union (EU) imposes regulations over PII of users located in the geographic region of the European Economic Area (EEA), which comprises various countries in Europe. The GDPR requires that Pll related technology complies with certain technical and organizational measures desired to secure and protect private user data. Similar laws and regulations are currently developing in the U.S., for example, the California Consumer Privacy Act (CCPA), which imposes similar regulations as the GDPR.

Accordingly, current monitoring technology provides security and PII risks to a user's data and creates exposure to regulations that impose technical requirements designed to protect such Pll and data.

For the foregoing reasons, there is a need for mmWave mapping systems and methods for generating one or more point clouds and determining one or more vital signs for defining a human psychological state. YANG ZHICHENG ET AL, in "Vital Sign and Sleep Monitoring Using Millimeter Wave", ACM TRANSACTIONS ON SENSOR NETWORKS, (20170424), vol. 13, no. 2, investigate such systems and methods.

WO 2021/067860 A1 describes a contactless sleep monitoring system that collects patient data from a plurality of sensors, including thermal, radar, and audio sensors.

### SUMMARY

The above problem and need is addressed by the millimeter wave (mmWave) mapping system of claim 1. Further advantageous aspects are set forth with the features of the subclaims. The present disclosure describes various aspects of millimeter wave (mmWave) technology, including mmWave sensors, configured for, among other things, eliminating or reducing a user's risk of Pll loss or unwanted exposure. The present disclosure describes mmWave mapping systems and methods for generating one or more point clouds and determining one or more vital signs for defining a human psychological state, which does not necessarily depend on conventional monitoring technology, including image sensors or other imaging technology.

In various aspects, the present disclosure describes mmWave mapping systems and methods that may be implemented as a standalone device, or as a multi-part system, each of which are configured to generate little or no PII data or information of a user. Additionally, or alternatively, the mmWave mapping systems and methods as disclosed herein may be combined with, or integrated with or into, existing monitoring technology (e.g., such as a LUMI device) and may be enabled and/or disabled by the user to provide monitoring, with or without, the generation or tracking of PII information, such as pictures, images, and/or video.

For example, the mmWave mapping systems and methods as described herein improves on conventional monitoring technologies in that the mmWave mapping systems and methods of the present disclosure provide technical solutions that have low or zero PII impact, have low maintenance requirements, and that are more durable and require less power and lower data storage given that no optic elements used and/or no images stored. In addition, the mmWave mapping systems and methods as described herein produce higher robustness and fidelity of raw data (e.g., point cloud data and vital signs as determined from mmWave waveforms) that are used to predict human psychological state(s) (e.g., sleep states) and related events in a more accurate manner compared with prior art technology. In one example, non-limiting use case, the mmWave mapping systems and methods can be implemented in a baby monitoring device or system to detect sleep quality of a person (e.g., a baby, a child, and/or an adult). Such baby monitoring device or system, implementing the systems and methods described herein, may monitor a baby or child without the use of imaging and/or PII (although, in some aspects, such data may be used to enhance tracking, monitoring, or artificial intelligence uses as described herein).

Other use cases are contemplated as well. For example, the mmWave mapping systems and methods as described herein may eliminate or reduce PII data loss and/or security issues, while also providing information about user habits, behavior, and/or user product use and/or consumption. For example, the mmWave mapping systems and methods may be used in various use cases including, by way of non-limiting example, automation of tasks, providing alerts for events (e.g., sleep detection or fall detection), and/or product replenishment. For example, the mmWave mapping systems and methods may be used to detect and identify products (e.g., a diaper in a physical space or container) and detect their fill level. For example, the mmWave mapping systems and methods may be used to detect loading of a diaper in a container or other storage area. In additional examples, the mmWave mapping systems and methods may be used to track user and simultaneously measure vital signs while the user is mobile within an environment or physical space. In various aspects, the mmWave mapping systems and methods are automated such that the mmWave mapping systems and methods are configured to passively monitor user behavior, with no user interaction needed.

The mmWave mapping systems and methods as described herein overcome prior art and conventional monitoring technology limitations in various ways. In particular, the mmWave mapping systems and methods as described herein use point cloud to identify a person's posture, a person's change in posture, and/or micro and/or macro movements of a person, each of which require little or no PII data. For example, in various aspects, point cloud data and/or vital sign measurements may comprise raw data forms that are fed into an mmWave API and/or machine learning model or algorithm to classify a human psychological state. Such human psychological state may, for example, define or represent a sleep quality of the person or user in a data abstract manner without revealing PII. In addition, mmWaves (e.g., electromagnetic waves referred to as "chirp signals" herein) are used in different and unique manners, for example, to both generate and/or determine a point cloud of a physical space (e.g., a room) and also measure vital signs of a person. In addition, the mmWave mapping systems and methods as described herein are uniquely configured with respect to frequency band, sweep bandwidth, antenna design, and/or spacing aspects so as to achieve desired measurements and determinations (e.g., vital signs and point cloud).

In addition, the mmWave mapping systems and methods as described herein are not necessarily bound to, or limited by, any specific location, configuration, or positioning within a physical space (e.g., positioning within or on furniture) in order to operate or function, e.g., to generate one or more point clouds and to determine one or more vital signs for defining a human psychological state. However, it is to be understood that location, configuration, or locationing within a physical space (e.g., positioning within or on furniture) is permissible, and in some use cases, desirable with respect to the mmWave mapping systems and methods described herein.

More specifically, as described herein, an mmWave mapping system is disclosed. In various aspects, the mmWave mapping system is configured to generate one or more point clouds and to determine one or more vital signs for defining a human psychological state. The mmWave mapping system comprises an mmWave sensor configured to transmit and receive one or more mmWave waveforms within a physical space. The mmWave mapping system further comprises one or more processors commutatively coupled to the mmWave sensor, wherein a digital signal processor of the one or more processors is configured to analyze the one or more mmWave waveforms. The mmWave mapping system further comprises an application (app) comprising a set of computing instructions. The set of computing instructions is configured for execution by the one or more processors, and when executed by the one or more processors, cause the one or more processors to generate, based on one or more mmWave waveforms of the mmWave sensor, a point cloud that maps at least a portion of the physical space. The point cloud comprises point cloud data defining a person or an object detected within the physical space. The set of computing instructions, when executed by the one or more processors, further cause the one or more processors to determine, based on the point cloud data, a posture of the person within the portion of the physical space. The set of computing instructions, when executed by the one or more processors, further cause the one or more processors to determine, based on the one or more mmWave waveforms of the mmWave sensor, one or more vital signs of the person. The set of computing instructions, when executed by the one or more processors, further cause the one or more processors to determine a human psychological state of the person as defined by the point cloud data and the one or more vital signs of the person. The set of computing instructions, when executed by the one or more processors, further cause the one or more processors to provide an electronic feedback representing the human psychological state as defined by the point cloud data and the one or more vital signs of the person.

In additional aspects, as described herein, an mmWave mapping method is disclosed for generating one or more point clouds and determining one or more vital signs for defining a human psychological state. The mmWave mapping method comprises generating, based on one or more mmWave waveforms of an mmWave sensor, a point cloud that maps at least a portion of a physical space, wherein the point cloud comprises point cloud data defining a person or an object detected within the physical space. The mmWave mapping method further comprises determining, based on the point cloud data, a posture of the person within the portion of the physical space. The mmWave mapping method further comprises determining, based on the one or more mmWave waveforms of the mmWave sensor, one or more vital signs of the person. The mmWave mapping method further comprises determining a human psychological state of the person as defined by the point cloud data and the one or more vital signs of the person. The mmWave mapping method further comprises providing an electronic feedback representing the human psychological state as defined by the point cloud data and the one or more vital signs of the person.

In still further aspects, a tangible, non-transitory computer-readable medium storing instructions for generating one or more point clouds and determining one or more vital signs for defining a human psychological state is disclosed. The instructions, when executed by one or more processors, cause the one or more processors to generate, based on one or more mmWave waveforms of an mmWave sensor, a point cloud that maps at least a portion of a physical space, wherein the point cloud comprises point cloud data defining a person or an object detected within the physical space. The instructions, when executed by one or more processors, further cause the one or more processors to determine, based on the point cloud data, a posture of the person within the portion of the physical space. The instructions, when executed by one or more processors, further cause the one or more processors to determine, based on the one or more mmWave waveforms of the mmWave sensor, one or more vital signs of the person. The instructions, when executed by one or more processors, further cause the one or more processors to determine a human psychological state of the person as defined by the point cloud data and the one or more vital signs of the person. The instructions, when executed by one or more processors, further cause the one or more processors to provide an electronic feedback representing the human psychological state as defined by the point cloud data and the one or more vital signs of the person.

In accordance with the above, and with the disclosure herein, the present disclosure describes improvements in computer functionality or in improvements to other technologies or technical fields at least because the disclosure describes, e.g., that the mmWave mapping systems and methods are configured to generate one or more point clouds and to determine one or more vital signs for defining a human psychological state. In various aspects, the mmWave system uses mmWave sensors that use less power and that are more durable than prior art systems that use images and videos that require camera based sensors. As a result the systems are able to operate more efficiently, and, in some aspects operate for a longer periods of time (e.g., for battery operated devices). In addition, the mmWave mapping systems and methods generate higher quality data than conventional devices, and eliminate user error, because a user can not remove or block accelerometer/sensors (as opposed to traditional camera based monitors requiring line-of-sight placement or locationing). Still further, given the mmWave electromagnetic wave use and related mapping, as described herein, the mmWave mapping systems and methods may provide accurate measurements even if line-of-sight to a user being monitored is obstructed. Yet further, the mmWave mapping systems and methods eliminate or reduce the PII generated for or by a user, so little or no PII data is able to be hacked or stolen via cyberattacks or otherwise.

In addition, the present disclosure relates to improvement to other technologies or technical fields at least because the present disclosure describes or introduces improvements to underlying computing devices, e.g. in some aspects, the mmWave mapping system is improved with an artificial intelligence learning model trained with vital sign data and point cloud data generated based on mmWave waveforms. The artificial intelligence learning model improves over prior art monitoring technologies at least because a computing device, e.g., mmWave mapping system or device, implementing or executing the artificial intelligence learning model is able to more accurately predict events or classify a representation of a human psychological state as defined by the point cloud data and the one or more vital signs of a person. Such classification and/or predictions may be used in the field of monitoring technology and/or tracking that was heretofore unavailable with previous devices or technologies in the field. In addition, the artificial intelligence learning model is further configured to be updated and evolve as it learns or receives data regarding additional human psychological states or related events and otherwise behavior(s), thereby further improving the predictive capabilities of the underlying mmWave mapping system.

The present disclosure includes applying certain aspects or features, as described herein, with, or by use of, a particular machine, e.g., an mmWave sensor configured to transmit and receive one or more mmWave waveforms within a physical space.

In addition, the present disclosure includes specific features other than what is well-understood, routine, or conventional activity in the field, by adding unconventional steps that confine the claims to a particular useful application, e.g., mmWave mapping systems and methods for generating one or more point clouds and determining one or more vital signs for defining a human psychological state.

Advantages will become more apparent to those of ordinary skill in the art from the following description of the preferred aspects which have been shown and described by way of illustration. As will be realized, the present aspects may be capable of other and different aspects, and their details are capable of modification in various respects. Accordingly, the drawings and description are to be regarded as illustrative in nature and not as restrictive.

### BRIEF DESCRIPTION OF THE DRAWINGS

The Figures described below depict various aspects of the system and methods disclosed therein. It should be understood that each Figure depicts an aspect of a particular aspect of the disclosed system and methods, and that each of the Figures is intended to accord with a possible aspect thereof. Further, wherever possible, the following description refers to the reference numerals included in the following Figures, in which features depicted in multiple Figures are designated with consistent reference numerals.

There are shown in the drawings arrangements which are presently discussed, it being understood, however, that the present aspects are not limited to the precise arrangements and instrumentalities shown, wherein:
FIG. 1 illustrates an example millimeter wave (mmWave) mapping system configured to generate one or more point clouds and to determine one or more vital signs for defining a human psychological state, in accordance with various aspects disclosed herein.
FIG. 2A illustrates further illustrates the example mmWave mapping system of FIG. 1 configured to generate one or more point clouds and to determine one or more vital signs for defining a human psychological state, in accordance with various aspects disclosed herein.
FIG. 2B illustrates an example visualization of a point cloud representative of and mapped to a physical space of FIG. 1.
FIG. 2C illustrates an example vital sign of a person detected within the physical space of FIG. 1.
FIG. 3 illustrates an example mmWave mapping method for generating one or more point clouds and determining one or more vital signs for defining a human psychological state, in accordance with various aspects disclosed herein.
FIG. 4 illustrates example electronic feedback of the mmWave mapping system of FIG. 1 and mmWave mapping method of FIG. 3 wherein the electronic feedback comprises visual feedback as displayed on a graphic user interface (GUI), in accordance with various aspects disclosed herein.
FIG. 5 further illustrates the example mmWave mapping system of FIG. 1 configured to generate one or more point clouds and to determine one or more vital signs for defining a human psychological state, in accordance with various aspects disclosed herein.

The Figures depict preferred aspects for purposes of illustration only. Alternative aspects of the systems and methods illustrated herein may be employed without departing from the principles of the invention described herein.

### DETAILED DESCRIPTION

According to one aspect, a millimeter wave (mmWave) mapping system is configured to generate one or more point clouds and to determine one or more vital signs for defining a human psychological state, the mmWave mapping system comprising:
an mmWave sensor configured to transmit and receive one or more mmWave waveforms within a physical space;
one or more processors commutatively coupled to the mmWave sensor, wherein a digital signal processor of the one or more processors is configured to analyze the one or more mmWave waveforms; and
an application (app) comprising a set of computing instructions, the set of computing instructions configured for execution by the one or more processors, and the set of computing instructions, when executed by the one or more processors, cause the one or more processors to:
   generate, based on one or more mmWave waveforms of the mmWave sensor, a point cloud that maps at least a portion of the physical space, wherein the point cloud comprises point cloud data defining a person or an object detected within the physical space,
   determine, based on the point cloud data, a posture of the person within the portion of the physical space,
   determine, based on the one or more mmWave waveforms of the mmWave sensor, one or more vital signs of the person,
   determine a human psychological state of the person as defined by the point cloud data and the one or more vital signs of the person, and
   provide an electronic feedback representing the human psychological state as defined by the point cloud data and the one or more vital signs of the person.

According to a further aspect, the point cloud comprises a two-dimensional (2D) point cloud, a three-dimensional (3D) point cloud, or a four-dimensional (4D) point cloud, wherein the 4D point cloud comprises a space dimension and a time value.

According to a further aspect, the human psychological state comprises one or more of: a sleep state, a restfulness state, a restlessness state, a stress state, a sadness state, or a happiness state.

According to a further aspect, the set of computing instructions, when executed by the one or more processors, further cause the one or more processors to:
generate a user-specific score defining a quality or quantity of the human psychological state as defined by one or more of: the point cloud data or the one or more vital signs of the person.

According to a further aspect, the user-specific score is rendered on a graphical user interface (GUI), and wherein the electronic feedback comprises visual feedback as displayed by the GUI.

According to a further aspect , the electronic feedback comprises a visual feedback, and wherein the visual feedback is configured for display on a graphic user interface (GUI).

According to a further aspect, the electronic feedback comprises an audio feedback, and wherein the audio feedback is configured for audio output on an audio device comprising one or more speakers.

According to a further aspect, the set of computing instructions, when executed by the one or more processors, further cause the one or more processors to:
generate a second point cloud that maps at least a portion of the physical space, wherein the second point cloud comprises second point cloud data defining the person or the object detected within the physical space, and wherein the second point cloud is generated at a second time period based on second one or more mmWave waveforms of the mmWave sensor, determine, based on the second point cloud data, a second posture of the person within the portion of the physical space,
determine, based on the second one or more mmWave waveforms of the mmWave sensor, a second one or more vital signs of the person,
determine the human psychological state as further defined by the second point cloud data and the second one or more vital signs of the person, and
provide a second electronic feedback representing the human psychological state as defined by the second point cloud data at the second time period and the second one or more vital signs of the person.

According to a further aspect, the set of computing instructions, when executed by the one or more processors, further cause the one or more processors to:
generate a state measurement value measuring a difference between a quality or quantity of the human psychological state between a first time period and the second time period.

According to a further aspect the mmWave mapping system is further comprising a second sensor communicatively coupled to the one or more processors,
wherein the one or more processors are configured to analyze a second data set generated by the second sensor, and
wherein the set of computing instructions, when executed by the one or more processors, further cause the one or more processors to:
determine, further based on the second data set, the human psychological of the person detected within the physical space.

According to a further aspect, the second sensor comprises one or more of: an environmental sensor configured to generate one or more of temperature data, audible data, or light data; a motion sensor configured to generate motion data; a camera configured to generate pixel data of a digital image; or a video sensor configured to generate pixel data comprising one or more image frames.

According to a further aspect, the set of computing instructions, when executed by the one or more processors, further cause the one or more processors to:
determine a chest displacement of the person based on the vital signs of the person.

According to a further aspect, the set of computing instructions, when executed by the one or more processors, further cause the one or more processors to:
determine a breathing rate or a heart rate based on the chest displacement.

According to a further aspect, the set of computing instructions, when executed by the one or more processors, further cause the one or more processors to:
determine at least one of: a type of the posture of the person, a volumetric size of the person, an area of the person, or a representation of a current posture of the person.

According to a further aspect, the mmWave mapping system is further comprising an artificial intelligence (AI) model,
wherein the Al model is trained with each of training point cloud data generated based on mmWave waveforms and vital sign data determined based on mmWave waveforms, and
wherein the Al model is configured to receive the point cloud data of the person and the one or more vital signs of the person as input and is further configured to output a sleep phase classification corresponding to the human psychological state as defined by the point cloud data of the person and the one or more vital signs of the person.

According to a further aspect, the Al model is further trained with a second training data set as generated by a second sensor,
wherein the Al model is configured to receive a new second data set generated by a second sensor as input and further configured to output the sleep phase classification further based on the new second data set.

According to a further aspect, the Al model is further configured to output a sleep quality classification, the sleep quality classification based on the sleep phase classification determined at a first time and a second sleep phase classification determined, by the Al model, at a second time.

According to a further aspect, the set of computing instructions, when executed by the one or more processors, further cause the one or more processors to:
generate a sleep deviation value based on the sleep phase classification determined at the first time and the second sleep phase classification determined at the second time.

According to a further aspect, the Al model is further trained with a plurality of user feedback responses defining sleep quality of respective users,
wherein the Al model is configured to receive one or more feedback responses of the person as input and output the sleep quality classification further based on the one or more feedback responses of the person.

According to a further aspect, the set of computing instructions, when executed by the one or more processors, further cause the one or more processors to:
generate a user-specific sleep recommendation based on the sleep quality classification.

According to a further aspect, the set of computing instructions, when executed by the one or more processors, further cause the one or more processors to:
update, based on the sleep quality classification, a state of a device communicatively coupled to the one or more processors.

According to a further aspect, the set of computing instructions, when executed by the one or more processors, further cause the one or more processors to:
receive an input specifying control of the electronic feedback, where the input causes one or more of: a starting or stopping of the electronic feedback, a frequency of the electronic feedback, or a pattern of providing or displaying the electronic feedback.

According to a further aspect, the object detected within the physical space is a non-person object.

According to a further aspect, at least one of the mmWave sensor or the one or more processors is positioned within or is in a proximity to a furniture within the physical space.

According to a further aspect, the furniture is a baby crib.

According to an aspect, a millimeter wave (mmWave) mapping method is provided for generating one or more point clouds and determining one or more vital signs for defining a human psychological state, the mmWave mapping method comprising:
generating, based on one or more mmWave waveforms of an mmWave sensor, a point cloud that maps at least a portion of a physical space, wherein the point cloud comprises point cloud data defining a person or an object detected within the physical space;
determining, based on the point cloud data, a posture of the person within the portion of the physical space;
determining, based on the one or more mmWave waveforms of the mmWave sensor, one or more vital signs of the person;
determining a human psychological state of the person as defined by the point cloud data and the one or more vital signs of the person; and
providing an electronic feedback representing the human psychological state as defined by the point cloud data and the one or more vital signs of the person.

According to a further aspect, the point cloud comprises a two-dimensional (2D) point cloud, a three-dimensional (3D) point cloud, or a four-dimensional (4D) point cloud, wherein the 4D point cloud comprises a space dimension and a time value.

According to a further aspect, the human psychological state comprises one or more of: a sleep state, a restfulness state, a restlessness state, a stress state, a sadness state, or a happiness state.

the set of computing instructions, when executed by the one or more processors, further cause the one or more processors to:
generate a user-specific score defining a quality or quantity of the human psychological state as defined by one or more of: the point cloud data or the one or more vital signs of the person.
the user-specific score is rendered on a graphical user interface (GUI), and wherein the electronic feedback comprises visual feedback as displayed by the GUI.

According to a further aspect, wherein the electronic feedback comprises a visual feedback, and wherein the visual feedback is configured for display on a graphic user interface (GUI).

According to a further aspect, the electronic feedback comprises an audio feedback, and wherein the audio feedback is configured for audio output on an audio device comprising one or more speakers.

According to a further aspect, the set of computing instructions, when executed by the one or more processors, further cause the one or more processors to:
generate a second point cloud that maps at least a portion of the physical space, wherein the second point cloud comprises second point cloud data defining the person or the object detected within the physical space, and wherein the second point cloud is generated at a second time period based on second one or more mmWave waveforms of the mmWave sensor, determine, based on the second point cloud data, a second posture of the person within the portion of the physical space,
determine, based on the second one or more mmWave waveforms of the mmWave sensor, a second one or more vital signs of the person,
determine the human psychological state as further defined by the second point cloud data and the second one or more vital signs of the person, and
provide a second electronic feedback representing the human psychological state as defined by the second point cloud data at the second time period and the second one or more vital signs of the person.

According to a further aspect, the set of computing instructions, when executed by the one or more processors, further cause the one or more processors to:
generate a state measurement value measuring a difference between a quality or quantity of the human psychological state between a first time period and the second time period.

According to a further aspect the mmWave mapping method is further comprising a second sensor communicatively coupled to the one or more processors,
wherein the one or more processors are configured to analyze a second data set generated by the second sensor, and
wherein the set of computing instructions, when executed by the one or more processors, further cause the one or more processors to:
   determine, further based on the second data set, the human psychological of the person detected within the physical space.

According to a further aspect, the second sensor comprises one or more of: an environmental sensor configured to generate one or more of temperature data, audible data, or light data; a motion sensor configured to generate motion data; a camera configured to generate pixel data of a digital image; or a video sensor configured to generate pixel data comprising one or more image frames.

According to a further aspect, the set of computing instructions, when executed by the one or more processors, further cause the one or more processors to:
determine a chest displacement of the person based on the vital signs of the person.

According to a further aspect, the set of computing instructions, when executed by the one or more processors, further cause the one or more processors to:
determine a breathing rate or a heart rate based on the chest displacement.

According to a further aspect, the set of computing instructions, when executed by the one or more processors, further cause the one or more processors to:
determine at least one of: a type of the posture of the person, a volumetric size of the person, an area of the person, or a representation of a current posture of the person.

According to a further aspect, the mmWave mapping method is further comprising an artificial intelligence (AI) model,
wherein the Al model is trained with each of training point cloud data generated based on mmWave waveforms and vital sign data determined based on mmWave waveforms, and
wherein the Al model is configured to receive the point cloud data of the person and the one or more vital signs of the person as input and is further configured to output a sleep phase classification corresponding to the human psychological state as defined by the point cloud data of the person and the one or more vital signs of the person.

According to a further aspect, the Al model is further trained with a second training data set as generated by a second sensor,
wherein the Al model is configured to receive a new second data set generated by a second sensor as input and further configured to output the sleep phase classification further based on the new second data set.

According to a further aspect, the Al model is further configured to output a sleep quality classification, the sleep quality classification based on the sleep phase classification determined at a first time and a second sleep phase classification determined, by the Al model, at a second time.

According to a further aspect, the set of computing instructions, when executed by the one or more processors, further cause the one or more processors to:
generate a sleep deviation value based on the sleep phase classification determined at the first time and the second sleep phase classification determined at the second time.

According to a further aspect, the Al model is further trained with a plurality of user feedback responses defining sleep quality of respective users,
wherein the Al model is configured to receive one or more feedback responses of the person as input and output the sleep quality classification further based on the one or more feedback responses of the person.

According to a further aspect, the set of computing instructions, when executed by the one or more processors, further cause the one or more processors to:
generate a user-specific sleep recommendation based on the sleep quality classification.

According to a further aspect, the set of computing instructions, when executed by the one or more processors, further cause the one or more processors to:
update, based on the sleep quality classification, a state of a device communicatively coupled to the one or more processors.

According to a further aspect, the set of computing instructions, when executed by the one or more processors, further cause the one or more processors to:
receive an input specifying control of the electronic feedback, where the input causes one or more of: a starting or stopping of the electronic feedback, a frequency of the electronic feedback, or a pattern of providing or displaying the electronic feedback.

According to a further aspect, the object detected within the physical space is a non-person object.

According to a further aspect, at least one of the mmWave sensor or the one or more processors is positioned within or is in a proximity to a furniture within the physical space.

According to a further aspect, the furniture is a baby crib.

According to an aspect a tangible, non-transitory computer-readable medium storing instructions is provided for generating one or more point clouds and determining one or more vital signs for defining a human psychological state, that when executed by one or more processors cause the one or more processors to:
generate, based on one or more mmWave waveforms of an mmWave sensor, a point cloud that maps at least a portion of a physical space, wherein the point cloud comprises point cloud data defining a person or an object detected within the physical space;
determine, based on the point cloud data, a posture of the person within the portion of the physical space;
determine, based on the one or more mmWave waveforms of the mmWave sensor, one or more vital signs of the person;
determine a human psychological state of the person as defined by the point cloud data and the one or more vital signs of the person; and
provide an electronic feedback representing the human psychological state as defined by the point cloud data and the one or more vital signs of the person.

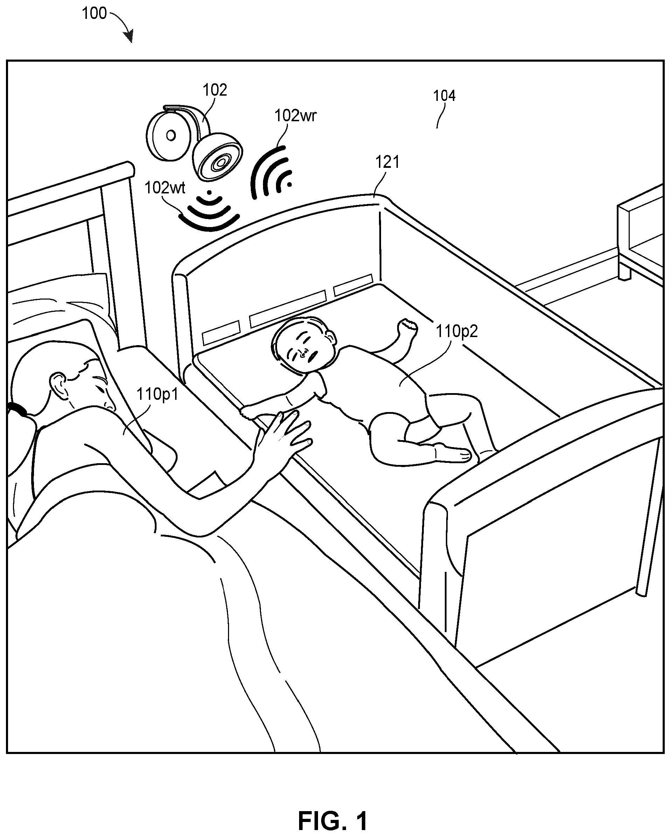

FIG. 1 illustrates an example millimeter wave (mmWave) mapping system 100 configured to generate one or more point clouds and to determine one or more vital signs for defining a human psychological state, in accordance with various aspects disclosed herein.

The example of FIG. 1 depicts a physical room or space (e.g., a physical space 104) comprising a first person 110p1 and a second person 110p2 (e.g., a mother and her child, respectively, as shown). Second person 110p2 (e.g., the child) is depicted sleeping in a posture different from that of the first person 110p1 (e.g., the mother), although it is to be understood that each of first person 110p1 and/or second person 110p2 may be positioned in various additional and/or different positions and/or postures which may be determined by the mmWave mapping systems and methods as described herein.

FIG. 1 further depicts an mmWave mapping device 102 of the mmWave mapping system 100 as mounted on a wall of the room (e.g., a physical space 104). It is to be understood, however, that mmWave mapping device 102 may be positioned in the room at other locations including, but not limited to, a desktop location, a floor location, a ceiling location, within furniture and/or walls, and/or other locations that otherwise position or configure mmWave mapping device 102 to transmit and receive mmWave waveforms as described herein.

In some embodiments, an object as detected within the physical space by mmWave mapping system 100 is a non-person object. For example, furniture 121 is an example of a non-person object positioned within physical space 104. As shown, second person 110p2 (e.g., the child) is depicted as resting or sleeping on furniture 121 positioned within physical space 104. In the example of FIG. 1, furniture 121 is a baby crib.

In various embodiments, mmWave mapping system 100 comprises one or more mmWave sensors configured to transmit and receive one or more mmWave waveforms within a physical space (e.g., physical space 104). In the example of FIG. 1, mmWave mapping device 102 may comprise one ore move mmWave sensors. An mmWave sensor comprises a radar-type sensor that transmits a predefined electromagnetic wave in a given spectrum. An mmWave sensor detects and/or senses electromagnetic waves bounced off of, or otherwise reflected from, objects in the room, similar to how sonar technology operates with respect to sound waves. In various embodiments, mmWave mapping device 102 and/or mmWave mapping system 100 incorporates and/or use mmWave sensors as manufactured by TEXAS INSTRUMENTS. However, it is to be understood, that mmWave sensors, as manufactured by or otherwise provided by, other manufactures may be used or incorporated in mmWave mapping device 102 and/or mmWave mapping system 100 for implementation of mmWave mapping systems and methods as described herein.

An mmWave spectrum of mmWave waveforms as transmitted and received by mmWave sensors may comprise a frequency range of 10 to 300 GHz. Although it is to be understood that other and/or different ranges (e.g., other high frequency ranges) are applicable to the systems and methods described herein. In addition, mmWaves waveforms are sometimes referred to, and may be referred to herein, as "chirp signals." For example, millimeter waves (i.e., "chirps") may transmitted from and received by an mmWave sensor, or otherwise mmWave transceiver, configured as part of mmWave mapping device 102.

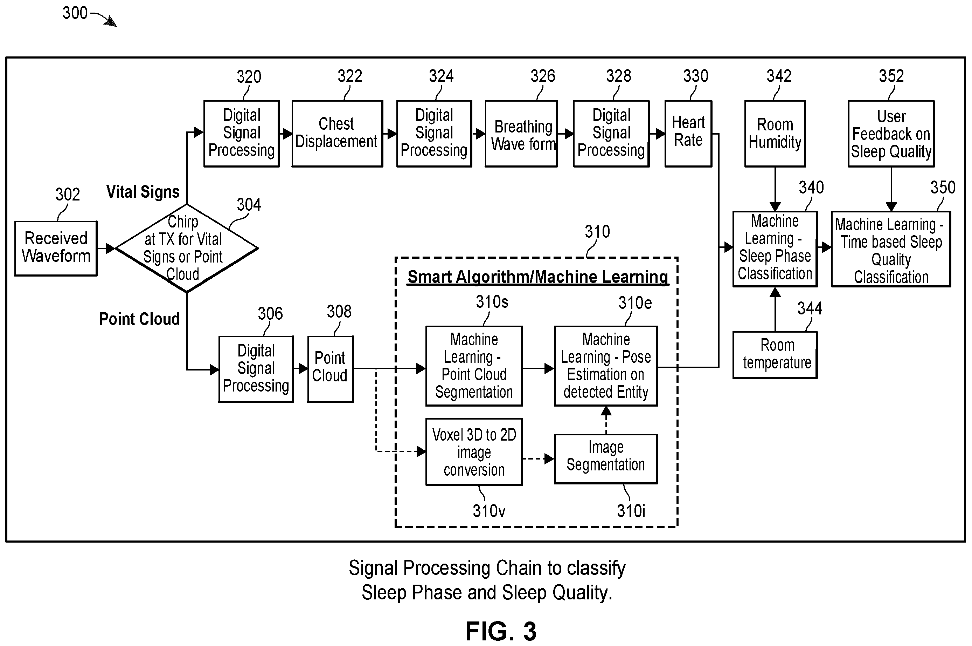

In the example of FIG. 1, transmit wave 102wt is an electromagnetic wave (i.e., "chirp" signal) as transmitted by an mmWave sensor of mmWave mapping device 102. Receive wave 102wr is an electromagnetic wave (i.e., "chirp" signal) as received by an mmWave sensor of mmWave mapping device 102. In some aspects, receive wave 102wr represents transmit wave 102wt at a different time, where transmit wave 102wt is bounced or otherwise propagated back or returned in physical space 104 (thereby becoming receive wave 102wr) after reflecting off of one or more objects (e.g., first person 110p1, second person 110p2, and/or furniture 121) in physical space 104. In other aspects, receive wave 102wr represents a different and additional electromagnetic wave, other than transmit wave 102wt, and based on a different and additional transmit wave (not shown), where the different and additional electromagnetic wave is bounced or otherwise propagated back or returned in physical space 104 (thereby becoming receive wave 102wr) after reflecting off of one or more objects (e.g., first person 110p1, second person 110p2, and/or furniture 121) in physical space 104. From this bouncing and/or reflection of mmWave waveforms transmitted and received by mmWave sensor, mmWave mapping device 102 may map or determine physical space 104 via generation of point cloud data. For example, a point cloud (e.g., point cloud 308) could be determined using digital signal processing (DSP) techniques (e.g., digital signal processing 306) on received mmWaves, e.g., as described with respect to Figure 3 or otherwise herein. Vital signs of persons (e.g., person 110p1 and/or person 110p2) may also be determined from the mmWave waveforms as described herein.

In various embodiments, mmWave mapping system 100 may be uniquely configured with respect to frequency band, sweep bandwidth, antenna design, and/or spacing aspects so as to achieve desired measurements and determinations (e.g., vital signs and point cloud). For example, taking Figure 1 as reference, the uniquely configured electromagnetic waves 102wt are transmitted in the target physical space 104. The reflected waves 102wr after interaction with animate objects such as 110p1 and 110p2 as well as inanimate objects such as 121 are received at the sensor. Generally, and without limitation, vital signs computation or generation, at least for some embodiments, can be described as: (1) using Digital Signal Processing (DSP) where distance is measured to respective objects; (2) Isolating torso displacement from the distance measured in step (1); (3) Computation of amplitude and phase; and (4) Using DSP techniques to compute breathing rate and heart rate.

Moreover, generally, and without limitation, point cloud computation or generation, at least for some embodiments, can be described as: (1) signal variance computation or determination; (2) computing direction of measurement using phase relation of antenna (multiple RX antennas); and (3) using advanced DSP techniques to compute one or more point clouds.

In various embodiments, mmWave mapping system 100 comprises one or more processors commutatively coupled to the mmWave sensor. A digital signal processor of the one or more processors is configured to analyze the one or more mmWave waveforms. For example, in FIG. 1, mmWave mapping device 102 may comprise one ore move processors commutatively coupled to the mmWave sensor(s), where the one or more processors include a digital signal processor configured to analyze one or more mmWave waveforms as transmitted and received by mmWave sensor(s) of mmWave mapping device 102 In some aspects, the processor may comprise an ARM 400Mz based processor. It is to be understood, however, that other processors, such as those from INTEL, NVIDIA, TEXAS INSTRUMENTS, or the like may be used in additional to, or in the alternative to, an ARM 400Mz based processor. The one or more processors may further be communicatively coupled, such as via an electronic bus, to one or more memories. The one or more memories may store computer instructions, mmWave data, waveforms, signals and/or other software, data, or signals for implementing the systems and methods as described herein.

In some aspects, each of the mmWave sensor(s), processor(s), and/or memorie(s) of mmWave mapping system 100 may be configured on a System-on-a-Chip (SoC) architecture. In such aspects, each of the mmWave sensor(s), processor(s), and/or memorie(s) may communicate electronically on circuitry or otherwise electronic architecture of the SoC, such as an electronic bus of the SoC. Such SoC architecture may be included with or at least as part of mmWave mapping device 102 or otherwise mmWave mapping system 100.

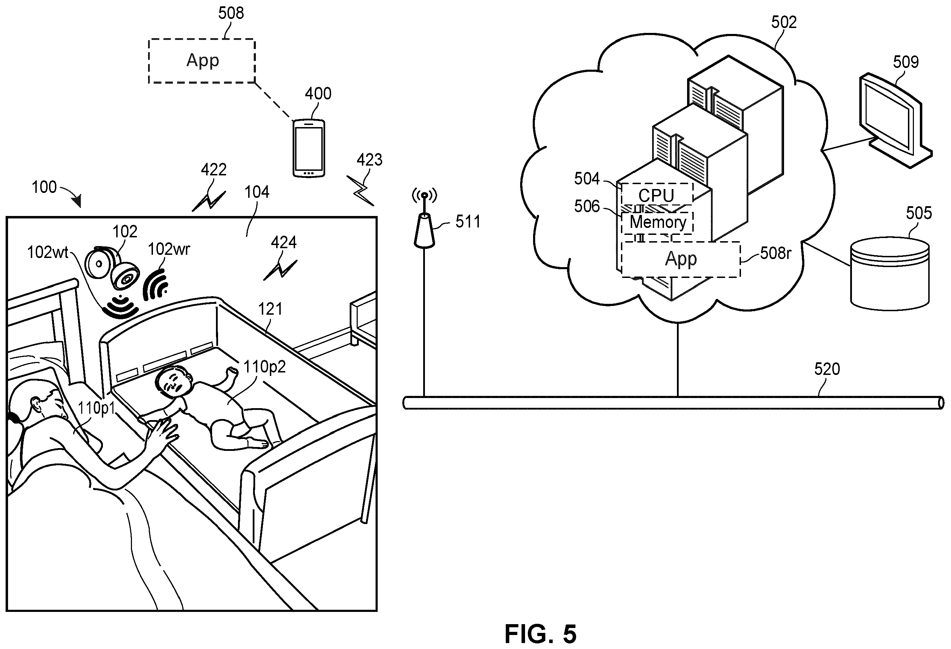

Additionally, or alternatively, the mmWave mapping system 100 may comprise remote server(s) that communicate with mmWave mapping device 102 via a computer network. For example, FIG. 5 further illustrates the example mmWave mapping system 100 of FIG. 1 configured to generate one or more point clouds and to determine one or more vital signs for defining a human psychological state, in accordance with various aspects disclosed herein. As shown for FIG. 5, mmWave mapping system 100 may comprise processor(s) and/or memorie(s), such as processor(s) and or memorie(s) of a server or cloud computing platform that communicate with an mmWave sensor remotely, such as over a computer network such as the Internet. For example, in various aspects mapping system 100 may comprise one or more servers 502, which may comprise multiple, redundant, or replicated servers as part of a server farm. In still further aspects, such server(s) 502 may be implemented as cloud-based servers, such as a cloud-based computing platform. For example, the server(s) 502 may be any one or more cloud-based platform(s) such as MICROSOFT AZURE, AMAZON AWS, or the like. The server(s) may include one or more processor(s) (e.g., CPU 504) as well as one or more computer memorie(s) (e.g., memory 506). Memorie(s) may include one or more forms of volatile and/or non-volatile, fixed and/or removable memory, such as read-only memory (ROM), electronic programmable read-only memory (EPROM), random access memory (RAM), erasable electronic programmable read-only memory (EEPROM), and/or other hard drives, flash memory, MicroSD cards, and others. Memorie(s) may store an operating system (OS) (e.g., MICROSOFT WINDOWS, LINUX, UNIX, etc.) capable of facilitating the functionalities, apps, methods, or other software as discussed herein. Memorie(s) (e.g., memory 506) may also store an application (app) 508r, which may comprise computing instructions as described herein for generating one or more point clouds and determining one or more vital signs for defining a human psychological state as described herein. Application (app) 508r may comprise at least a portion, such as a remote portion, of the computing instructions, where a remaining or local portion (e.g., app 508 implemented on mobile device 400) executes in communication with app 508r across a computer network 520.

Server(s) 502 may further include a communication component configured to communicate (e.g., send and receive) data via one or more external/network port(s) to one or more networks or local terminals, such as computer network 520 and/or terminal 509 (for accessing mmWave data and/or point cloud data) described herein. In some embodiments, imaging server(s) 502 may include a client-server platform technology such as ASP.NET, Java J2EE, Ruby on Rails, Node.js, a web service or online API, responsive for receiving and responding to electronic requests. Server(s) 502 may implement the client-server platform technology that may interact, via the computer bus, with the memories(s) 506 (including the applications(s), component(s), API(s), data, etc. stored therein) and/or database 505 to implement or perform the machine readable instructions, methods, processes, elements or limitations, as illustrated, depicted, or described for the various flowcharts, illustrations, diagrams, figures, and/or other disclosure herein.

Server(s) 502 may also be communicatively coupled to database 505, which may store mmWave data, point cloud data, and/or other data as described herein. Such mmWave data, point cloud data, and/or other data as described herein may be transmitted between app 508 and app 508r, where app 508r can store and/or access such information from memory 506 and/or database 505. Memories (e.g., memory 506) may store machine readable instructions, including any of one or more application(s) (e.g., app 508r comprising a set of computing instructions as described herein), one or more software component(s), and/or one or more application programming interfaces (APIs), which may be implemented to facilitate or perform the features, functions, or other disclosure described herein, such as any methods, processes, elements or limitations, as illustrated, depicted, or described for the various flowcharts, illustrations, diagrams, figures, and/or other disclosure herein. For example, at least some of the applications, software components, or APIs may be configured to facilitate their various functionalities discussed herein. It should be appreciated that one or more other applications may be envisioned and that are executed by the processor(s) (e.g., CPU 504).

In addition, the one or more processors (e.g., CPU 504) as described herein may be connected to the memorie(s) (e.g., memory 506) via a computer bus responsible for transmitting and/or receiving electronic data, data packets, or otherwise electronic signals to and from the processor(s) and memorie(s) in order to implement or perform the machine readable instructions, methods, processes, elements or limitations, as illustrated, depicted, or described for the various flowcharts, illustrations, diagrams, figures, and/or other disclosure herein.

processor(s) (e.g., CPU 504) may include, or interact with, one or more transceivers (e.g., WWAN, WLAN, and/or WPAN transceivers) functioning in accordance with IEEE standards, 3GPP standards, or other standards, and that may be used in receipt and transmission of data via external/network ports connected to computer network 520. In some aspects, the computer network 520 may comprise a private network or local area network (LAN). Additionally, or alternatively, the computer network may comprise a public network such as the Internet.

Server(s) 502 may further include or implement an operator interface configured to present information to an administrator or operator and/or receive inputs from the administrator or operator. As shown in FIG. 5, an operator interface may provide a display screen (e.g., via terminal 509). Server(s) 502 may also provide I/O components (e.g., ports, capacitive or resistive touch sensitive input panels, keys, buttons, lights, LEDs), which may be directly accessible via, or attached to, server(s) 502 or may be indirectly accessible via or attached to terminal 509. According to some embodiments, an administrator or operator may access the server 502 via terminal 509 to review information, make changes, input data, and/or perform other functions.

As described herein, in some embodiments, server(s) 502 may perform the functionalities as discussed herein as part of a "cloud" network or may otherwise communicate with other hardware or software components within the cloud to send, retrieve, or otherwise analyze data or information described herein, including communication between apps 508 and 508r.

In general, a computer program or computer based product, application (app), or code (e.g., the application (app) of the mmWave mapping system and/or methods or computing instructions described herein) may be stored on a computer usable storage medium, or tangible, non-transitory computer-readable medium (e.g., random access memory (RAM), an optical disc, a universal serial bus (USB) drive, or the like) having such computer-readable program code or computer instructions embodied therein, wherein the computer-readable program code or computer instructions may be installed on or otherwise adapted to be executed by the processor(s) and/or processors of mobile devices (e.g., mobile device 400) (e.g., working in connection with the respective operating system) to facilitate, implement, or perform the machine readable instructions, methods, processes, elements or limitations, as illustrated, depicted, or described for the various flowcharts, illustrations, diagrams, figures, and/or other disclosure herein. In this regard, the program code may be implemented in any desired program language, and may be implemented as machine code, assembly code, byte code, interpretable source code or the like (e.g., via Golang, Python, C, C++, C#, Objective-C, Java, Scala, ActionScript, JavaScript, HTML, CSS, XML, etc.).

As shown in FIG. 5, server(s) 502 are communicatively connected, via computer network 520 to mobile device 400 via base station 511. In some embodiments, base station 511 comprises a cellular base station, such as a cell tower, communicating to mobile device 400 via wireless communication 423 based on any one or more of various mobile phone standards, including NMT, GSM, CDMA, UMMTS, LTE, 5G, or the like. Additionally, or alternatively, base station 511 may comprise routers, wireless switches, or other such wireless connection points communicating to mobile device 400 via wireless communication 424 based on any one or more of various wireless standards, including by non-limiting example, IEEE 802.11a/b/c/g (WIFI), the BLUETOOTH standard, or the like.

Mobile device 400 may comprise a mobile device and/or client device for accessing and/or communications with server(s) 502. Such mobile devices may comprise one or more mobile processor(s). In various embodiments, mobile device 400 comprises a mobile phone (e.g., a cellular phone), a tablet device, a personal data assistance (PDA), or the like, including, by non-limiting example, an APPLE iPhone or iPad device or a GOOGLE ANDROID based mobile phone or table. Mobile device 400 may communicate directly (e.g., via wireless communication 422, such as WIFI or BLUETOOTH) with mmWave mapping device 102. In addition, mmWave mapping device 102 may communicate directly with base station 511 via wireless communication 424, such as WIFI or BLUETOOTH. In this way, connectivity among mobile device 400, mmWave mapping device 102, and base station 511 provide communication between mmWave mapping device 102, mobile device 400, and server(s) 502 for sending and receiving data, such as mmWave data and/or points clouds for determining one or more vital signs for defining a human psychological state a described herein.

With reference to FIGs. 1 and 5, it is to be understood that additional or alternative configurations and/or positions of the mmWave sensor(s), processor(s), and/or memorie(s) of mmWave mapping system 100 are contemplated herein such that these components are configured and/or positioned so as to be electronically and communicatively coupled with respect to one another for generating one or more point clouds and determining one or more vital signs for defining a human psychological state, in accordance with various aspects disclosed herein

For example, in some embodiments, at least one of the mmWave sensor or the one or more processors (e.g., of mmWave mapping device 102), may be positioned within or is in a proximity to a furniture within the physical space. In some example aspects, at least one of the mmWave sensor or the one or more processors (e.g., of mmWave mapping device 102) may be positioned within or is in a proximity to furniture 121 (e.g., a baby crib), as depicted for FIG. 1. It is to be understood, however, that other furniture or non-person objects are contemplated herein, where, for example such furniture and/or non-person objects may comprise desks, beds, night stands, walls and/or ceilings (such as inside of partially inside of walls and/or ceiling, etc.), and the like, where the mmWave sensor(s) and/or the one or more processors may be positioned within (e.g., fully or partially within) or in a proximity to the furniture and/or non-person objects.

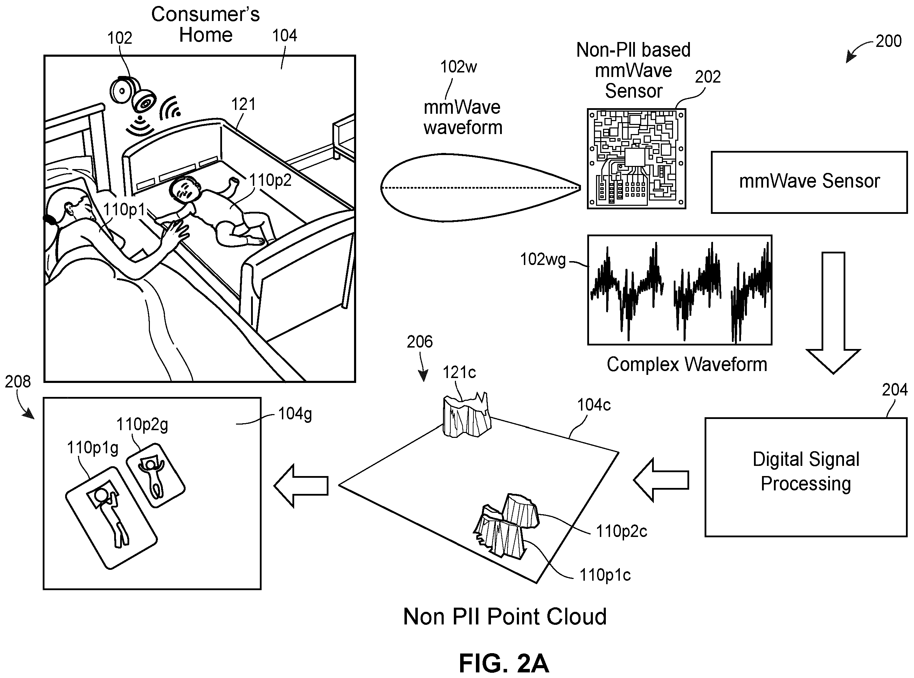

FIG. 2A further illustrates the example mmWave mapping system 100 of FIG. 1 configured to generate one or more point clouds and to determine one or more vital signs for defining a human psychological state, in accordance with various aspects disclosed herein. FIG. 2A depicts a schematic or flow diagram of operation of example mmWave mapping system 100.

As depicted in FIG. 2A, an mmWave sensor 202 (e.g. as configured within or as part of mmWave mapping device 102) transmits and receives mmWave waveforms (e.g., wave 102w) within physical space 104. Wave 102 represents electromagnetic waves (i.e., chirp signals), for example transmit wave 102wt and/or receive wave 102wr, as described herein for FIG. 1. As depicted for FIG. 2A, and in various aspects, electromagnetic waves, such as any of wave 102w, transmit wave 102wt, and/or receive wave 102wr, may be transmitted and/or received in beam form (e.g., a targeted or directed electromagnetic waves) and/or broadcast form (e.g., omnidirectional or spherical wave transmission and reception). In some aspects, waveform type or shape (e.g., whether beam and/or omnidirectional) may be set or configured by a user or otherwise for mmWave mapping device 102 and/or mmWave mapping system 100. In some embodiments, a multiple-input and multiple-output (MIMO) antenna may be used to transmit any one or more of the electromagnetic waves described herein, including for example such as any of wave 102w, transmit wave 102wt, and/or receive wave 102wr. Such MIMO antenna may be configured as part of and/or within mmWave mapping device 102, as illustrated for Figure 1. It should be noted, however, that other additional or alternative antenna(s) capable of transmitting and receiving electromagnetic waves (i.e., chirp signals) may be used as well.

The mmWave sensor 202 may detect or sense persons or otherwise objects in a physical space, for example, as described for FIG. 1. In the example of FIG. 2A, first person 110p1 (e.g., a mother) and second person 110p2 (e.g., a child) may be detected or sensed within physical space 104 by mmWave sensor 202. As shown for FIG. 2A, processor(s) of mmWave mapping device 102 or otherwise of mmWave mapping system 100 may implement digital signal processing 204 of the received waveforms (e.g., 102w and/or receive wave 102wr). Digital signal processing 204 comprises execution of computing instructions, as described herein, to analyze mmWave waveforms as data input of raw or complex waveforms 102wg. In various aspects, mmWave sensor 202 receives reflected electromagnetic wave(s) (e.g., receive wave 102wr) of person(s) and/or object(s) to determine range, velocity, direction, and/or otherwise detection or presence of person(s) and/or object(s) in a physical space (e.g., physical space 104). The detection or sensing of persons or otherwise objects is based on processing and analyzing of raw waveforms (e.g., any of wave 102w and/or receive wave 102wr) as received by mmWave sensor 202. For example, an intensity or value, or change in intensity or value over time, of waveforms analyzed by one or more processors of mmWave mapping device 102 (e.g., a digital signal processor) may indicate detection or presence persons or otherwise objects within a physical space (e.g., physical space 104).

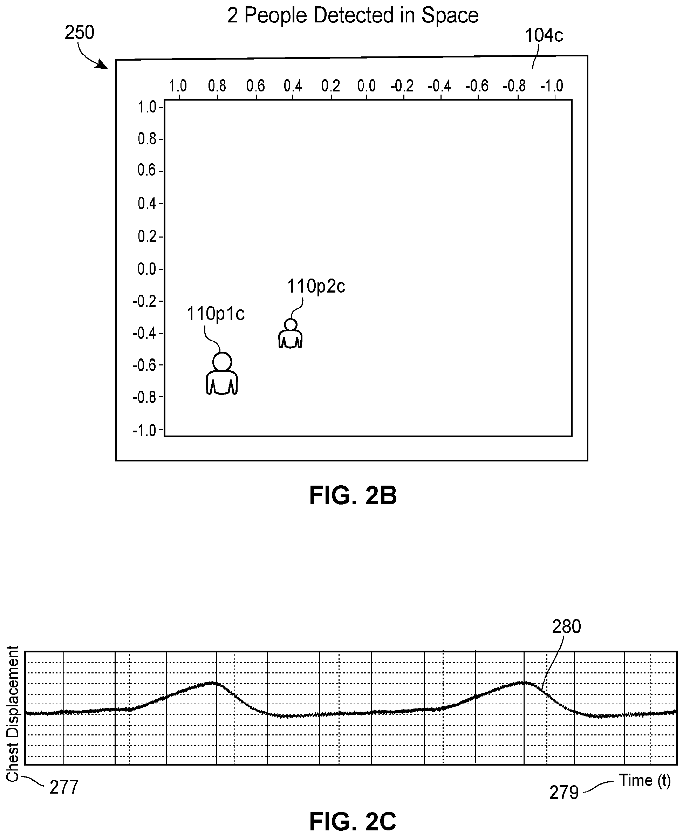

In some aspects, intensity or value, or change in intensity or value over time, of waveforms may be graphically represented, for example, as illustrated by complex waveforms 102wg and/or FIG. 2C. For example, a slope ramping up over time (e.g., indicating intensity or value surge) as determined from mmWave waveforms analyzed by processor(s) indicates detection or a person or object, or a portion of a person or object. In some aspects, such a detection may trigger an action or be indicative of an event. It is to be understood, that waveforms (i.e., chirp signals) may be specific to a particular application. For example, mmWave waveforms may be used to detect or determine vital signs of person(s) in a physical space (e.g. physical space 104). In other embodiments, mmWave waveforms may be used to generate or determine point clouds and/or point cloud data. Such mmWave waveforms, as detected, may be triggers for various use cases, e.g., detecting persons or objects in a room, or detecting the presence or absence of a product in a space (e.g., the presence or absence of a diaper in a container or otherwise storage location).

In some aspects, digital signal processing 204 of mmWave waveforms (i.e., chirp signals) produces outputs. For example, digital signal processing 204 may be implemented by processor(s) of mmWave mapping device 102 or otherwise mmWave mapping system 100 to generate or output a point cloud 206 having corresponding point cloud data.

Generally, as described herein, a point cloud may comprise a two-dimensional (2D) point cloud, a three-dimensional (3D) point cloud, or a four-dimensional (4D) point cloud. A 4D point cloud comprises a space dimension and a time value. In various aspects, a point cloud and/or its respective point cloud data may be used by processor(s) of mmWave mapping system 100 to measure or determine posture of a person in a physical space, such as a sleeping posture. Point clouds and related data may also be used to determine, detect, or track people in the physical space, which may include, e.g., tracking one or more people a room (e.g., physical space 104).

A point cloud may be generated by processor(s) of mmWave mapping device 102 or otherwise mmWave mapping system 100 based on digital signal processing 204 of mmWave waveforms. In particular, the range, velocity, direction, as determined from the mmWave waveforms may be used by processor(s) (e.g., a digital signal processor) to generate a point cloud of a person or object within a physical space. A point cloud defines a set of coordinates in 2D (e.g., x and y coordinates) and/or 3D space (e.g., x, y, and z coordinates), and may further define an outline or otherwise presence of a person or object within that 2D and/or 3D space. A 4D detection adds a time value for when such detection or outline occurred within the 2D and/or 3D space. While the outline or presence of person or object within a 2D and/or 3D space allows mmWave mapping system 100 to determine a person is detected, such outline or presence is secure because the mmWave mapping system 100 keeps PII of the person(s) detected protected in that the outline or presence does not provide enough information to allow identification of the person(s) detected, but, at the same time, allows analysis and determination of positioning and/or otherwise presence of an a person or object in 2D, 3D, and/or 4D space.

With reference to FIG. 2A, point cloud 206 is depicted as a 3D point cloud. As illustrated, outline 110p1c defines a representation, within point cloud 206, of person 100p1 (e.g., the mother) within physical space 104 as represented by point cloud space 104c. Similarly, outline 110p2c defines a representation, within point cloud 206, of person 100p2 (e.g., the child) within physical space 104 as represented by point cloud space 104c. Still further, outline 121c defines a representation, within point cloud 206, of a non-person object (e.g., furniture 121 or a portion thereof) within physical space 104 as represented by point cloud space 104c. Each of outline 110p1c, outline 110p2c, and outline 121c are generated from processor(s) (e.g., a digital signal processor, micro controller, microprocessor, or the like) analyzing mmWave waveforms (e.g., chirp signals) as transmitted and received by mmWave sensor 202 as described herein. In addition, as shown, each of outline 110p1c, outline 110p2c, and outline 121c represent a non-Pll based point cloud that security represents person 100p1's and person 110p2's respective PII.

FIG. 2B illustrates an example visualization of a point cloud 250 representative of and mapped to a physical space 104 of FIG. 1. In particular, FIG. 2B represents a 2D representation of point cloud 206 of FIG. 2A. In some aspects, point cloud 250 is a top-down representation of point cloud 206, that may further obscure the PII of person 100p1 (e.g., the mother) and person 110p2 (e.g., the child) detected in physical space 104 represented by point cloud space 104c.

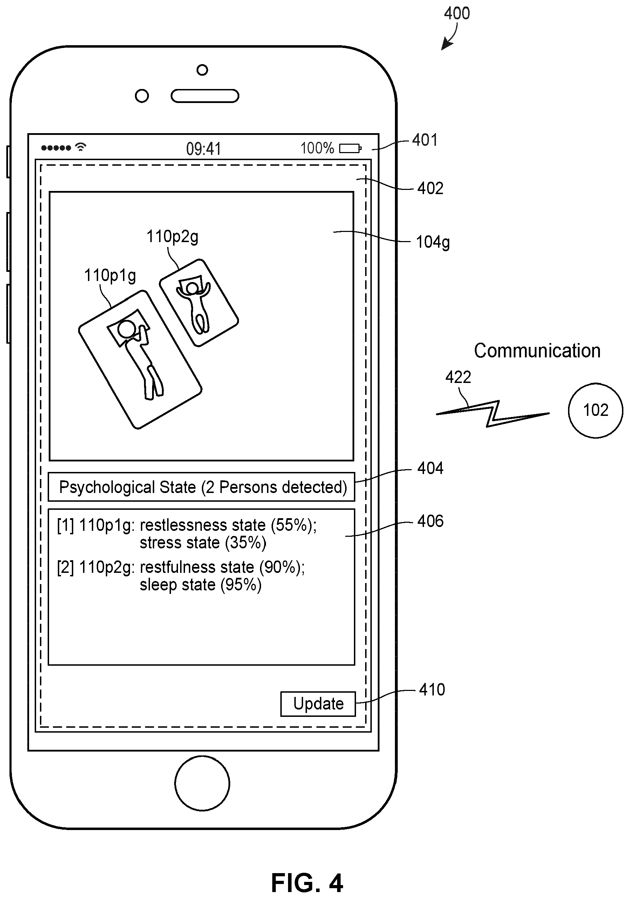

In some aspects, point cloud 206 of FIG. 2A and/or point cloud 250 of FIG. 2B, in either 2D, 3D, or 4D form may be rendered on a display or GUI, e.g., as described for FIG. 4 herein.

The mmWave waveforms, as described herein, may also be used to determine or detect vital signs of persons. For example, such vital signs may include, but are not limited to, heart rate, breathing rate, etc. Such vital signs may be measured by analysis of mmWave waveforms to detect or determine a person's posture, a person's change in posture, and/or movement moves of person (e.g., micro and/or micro movement) of body parts or portions of persons.

FIG. 2C illustrates an example vital sign 275 (e.g., breathing pattern) of a person detected within the physical space of FIG. 1. Vital sign 275 is graphically depicted having a time measurement 279 and a chest displacement measurement 277. Time measurement 279 may be recorded in, but is not limited to, units of seconds, milliseconds, or the like. Chest displacement measurement 277 may be measured in intensity values, amplitude(s), distance measurements (e.g., distance from mmWave sensor 202 to a person or object), or as otherwise described herein. For example, digital signal processing 204 may be implemented to compute chest displacement depicted for FIG. 2C, or other vital signals, including for example, but not limited to, breathing rate and/or heart rate. For example, in some aspects, a set of computing instructions, when executed by the one or more processors (e.g., a digital signal processor, micro controller, microprocessor, or the like), may cause the one or more processors to determine, based on at least one of the point cloud data or the vital signs of the person, a chest displacement of the person. In specific aspects, the set of computing instructions, when executed by the one or more processors, further cause the one or more processors to determine a breathing rate or a heart rate based on the chest displacement (e.g., differences in measurements and/or positioning of chest displacement of a person).

As shown in the example of FIG. 2C, amplitude 280 are recorded over time, illustrating a person (e.g., person 110p1, the mother's, or person 110p2, the child's) breathing pattern. In this way, the vital sign (e.g., breathing pattern) may be detected and/or recorded by mmWave mapping system 100.

In some aspects, vital sign 275 may comprise a graphic electronic feedback that may be rendered on a display or GUI, e.g., as described for FIG. 4 herein.

FIG. 2A also illustrates graphical depiction 208, which represents a high level abstraction of person 110p1 (e.g., the mother) and person 110p2 (e.g., the child) and their related positions in physical space 104 as represented by graphical physical space 104g. For example, graphical depiction 208 illustrates graphic 110p1g representing person 110p1 (e.g., the mother) and graphic 110p2p representing person 110p2 (e.g., the child). Graphic 110p1g and graphic 110p2g may be rendered or represented as icons or otherwise graphics in graphical physical space 104g that are similar to the real or physical persons or objects in physical space 104. In additional aspects, graphics or icons of person or objects may be rendered or depicted within graphical physical space 104g having specific positions, locations, postures, indications, or otherwise graphics to represent or correspond to real-life specific positions, locations, postures, indications in physical space 104.

For example, as shown in FIG. 2A, graphic 110p1g is rendered as an icon of an adult sized person with a posture of sleeping on his or her side facing a given direction (e.g., eastward) within graphical physical space 104g that mirrors or corresponds to the person 110p1's (e.g., the mother's) real-life position and/or posture within physical space 104 as detected by mmWave sensor 202 or otherwise mmWave mapping system 100.

As a further example, as shown in FIG. 2A, graphic 110p2g is rendered as an icon of a child sized person with a posture sleeping on his or her back facing a given direction (upwards) within graphical physical space 104g that mirrors or corresponds to the person 110p2's (e.g., the child's) real-life position and/or posture within physical space 104 as detected by mmWave sensor 202 or otherwise mmWave mapping system 100.

Generally, graphical depiction 208 represents a high level, graphical illustration of persons (e.g., persons 110p1 and 110p2) within physical space 104 that accurately portrays posturing and positions, and at the same time protects and securely provides such information without compromising PII of the persons because the graphics or icons are high level representations of the information.

In some aspects, graphical depiction 208, comprising an electronic feedback representing human physiological states(s) may be rendered on a display or GUI, e.g., as described for FIG. 4 herein.

FIG. 3 illustrates an example mmWave mapping method 300 for generating one or more point clouds and determining one or more vital signs for defining a human psychological state, in accordance with various aspects disclosed herein. In some aspects, mmWave mapping method 300 may be implemented with one or more artificial intelligence (AI) models, including, for example, a sleep classification model, as described herein below. For example one or more Al models may be trained on mmWave waveforms (e.g., representing vital signs or point cloud data), and other data (e.g., body mass index (BMI) of persons in the room, age, and/or height or the like, as entered by a user via graphic user interface such as desc2cribed for FIG. 3 (block 352) and/or FIG. 4) to determine or detect persons or objects within a physical space, and/or their attributes (e.g., body mass index (BMI) of persons in the room) and/or to generate vital signs or indicate events or classification (e.g., sleep quality) described herein.

The mmWave mapping method 300 may be implemented as part of an application (app) comprising a set of computing instructions configured for execution by one or more processors (e.g., one or more processors of mmWave mapping device 102 or otherwise mmWave mapping system 100). The one or more processors, may be in some aspects, one or more processors of a mobile device (such as an APPLE IPHONE or a GOOGLE ANDROID device), e.g., mobile device 400 of FIG. 4, where the app is a mobile app implementing the computing instructions in a native programming language (e.g., Objective-C, SWIFT, or JAVA). In other aspects, the one or more processors, may be one or more processors of mmWave mapping device 102 and/or server(s) communicatively coupled to mmWave mapping device 102 and/or a mobile device as described herein for FIG. 4.

At block 302, mmWave waveforms may be received by processors (e.g., a digital signal processor) and processed at various blocks of mmWave mapping method 300 (e.g., blocks 302-352).

At block 304, mmWave mapping method 300 may comprise determining whether an mmWave waveform (i.e., chirp signal) will be processed to determine a vital sign and/or point cloud. In this way, mmWave mapping method 300 may use or define different chirp signal categories for each of vital sign(s) measurement and point cloud measurement.

At block 306, mmWave mapping method 300 comprises implementing or executing the set of computing instructions on or by the one or more processors to cause the one or more processors to, at block 308, generate, based on one or more mmWave waveforms of the mmWave sensor, a point cloud (e.g., point cloud 206 and/or 250) that maps at least a portion of the physical space (e.g., physical space 104). The point cloud (e.g., point cloud 206 and/or 250) comprises point cloud data defining a person or an object (e.g., person 110p1, person 110p2, and/or further 121) detected within the physical space (e.g., physical space 104). Other non-person objects, such as fans, etc., may be detected as well. More generally, the point cloud data may include or be used to detect or determine unique objects, including human or inanimate objects, to track movements within a physical space (e.g., physical space 104), identify behavior (e.g., postures or vital sign activities, such as breathing), track product consumption (e.g., diaper usage), and/or identify events such as a person falling, etc. within the physical space (e.g., physical space 104).

mmWave mapping method 300 comprises computing instructions, that when executed by the one or more processors, further cause the one or more processors to determine at least one of a type of the posture of a person, a volumetric size of the person (e.g., height and/or width of a person), an area of the person, or a representation of a current posture of the person. For example at blocks 306 and 308, mmWave mapping method 300 may comprise determining, based on the point cloud data, a posture of the person within the portion of the physical space. As described for FIG. 2A, a person's posture, a person's change in posture, and/or micro and/or macro movements of a person, within a physical space (e.g., physical space 104) may be detected or determined from 2D, 3D, and/or 4D point cloud measurements.

In some aspects, at block 310, mmWave mapping method 300 comprises implementation of Al models or algorithms, including machine learning, and preprocessing to determine or detect persons or objects within physical space 104. For example, block 310 comprises Al preprocessing steps of point cloud segmentation 310s to determine shapes within a set of point cloud data as determined with respect to blocks 306 and 308. Such Al preprocessing may further comprise, at block 310v, voxel 3D to 2D image conversion (in aspects involving 2D point cloud data, for example as described for FIGS. 2A and 2B), where 3D points in a 3D point cloud (e.g., point cloud 206) are converted to 2D points in a 2D point cloud (e.g., point cloud 250). At block 310i, the 2D images within a point cloud space (e.g., point cloud space 104c of either of FIGS. 2A or 2B) may be segmented and identified as outline images or graphics (e.g., any one or more of outline 110p1c, outline 110p2c, and outline 121c in point cloud space 104c). Further, at block 310e a trained machine learning model, as trained on image segmentation data (e.g., outline 110p1c, outline 110p2c, and/or outline 121c in point cloud space 104c) and/or point cloud segmentation data may be used to determine, predict, and/or classify a user's posture, pose, and/or position within the physical space (e.g., physical space 104 as represented by point cloud space 104c). The output of the model, for example, may comprise defined outline images or graphics (e.g., any one or more of outline 110p1c, outline 110p2c, and outline 121c in point cloud space 104c) that may be provided or output to block 340 for further analysis and implementation as described herein.

At blocks 320-330, mmWave mapping method 300 comprises determining, based on the one or more mmWave waveforms of the mmWave sensor, one or more vital signs of the person (e.g., person 110p1 or person 110p2). For example, each of blocks 320, 324, and 328 comprise computing instructions for determining chest displacement (at block 322), breathing waveform (block 326), and related heart rate (block 330). Such processing and determinations may be determined, for example, as described for FIGs. 2A and 2C or otherwise herein. In some embodiments, for example, a heart rate (block 330) may be determined or extrapolated based on a user's BMI information (e.g., from data provided by the user, for example as described for FIG., 3, block 352 and/or FIG. 4) together with mmWave data corresponding to the user's current chest displacement (block 322) and breathing rate, amplitude, and/or intensity as described for FIGs. 2A and 2C or otherwise herein. However, it is to be understood, that in various embodiments, mmWave data alone (without user provided data) may be used to determine heart rate and/or breathing rate.

At block 340, mmWave mapping method 300 comprises determining a human psychological state of the person as defined by the point cloud data and the one or more vital signs of the person. In some aspects, additional sensors and related data are used to determine a human psychological state (e.g., sleep phase classification) of a person (e.g., person 110p1). For example, in some aspects a second sensor may be communicatively coupled to one or more processors (e.g., processor(s) of mmWave mapping device 102) that are configured to analyze a second data set generated by the second sensor. The second data set may comprise environmental data of physical space 104 including temperature data (e.g., block 342 via a thermometer sensor), sound (e.g., via a microphone), light (e.g., via a photocell or camera), humidity (e.g., block 344 via a relative humidity sensor), motion (e.g., via a motion sensor), and/or video (e.g., via a video camera). In such aspects, mmWave mapping system 100 may further comprise an environmental sensor configured to generate one or more of temperature data, audible data, or light data; a motion sensor configured to generate motion data; a camera configured to generate pixel data of a digital image; or a video sensor configured to generate pixel data comprising one or more image frames. Still further, in such aspects, the computing instructions, when executed by the one or more processors (e.g., one or more processors of mmWave mapping system 100), further cause the one or more processors to determine, further based on the second data (e.g., the environment data), the human psychological of the person detected within the physical space (e.g., physical space 104).

In other aspects, artificial intelligence (Al), including trained Al models, is used to determine vital sign(s), generate point clouds or related information, or generate or determine sleep phase classifications. An Al model, including, for example, a sleep classification model of block 340, is trained using a supervised or unsupervised machine learning program or algorithm. The machine learning program or algorithm may employ a neural network, which may be a convolutional neural network, a deep learning neural network, or a combined learning module or program that learns in two or more features or feature datasets (e.g., such as mmWave waveform signals or data including vital sign data and/or point cloud data based that may be determined therefrom) in a particular area of interest. The machine learning programs or algorithms may also include natural language processing, semantic analysis, automatic reasoning, regression analysis, support vector machine (SVM) analysis, decision tree analysis, random forest analysis, K-Nearest neighbor analysis, naïve Bayes analysis, clustering, reinforcement learning, and/or other machine learning algorithms and/or techniques. In some aspects, the artificial intelligence and/or machine learning based algorithms may be included as a library or package executed on server(s) 502. For example, libraries may include the GOOGLE TENSORFLOW based library, the PYTORCH library, and/or the SCIKIT-LEARN Python library.

Machine learning may involve identifying and recognizing patterns in existing data (such as mmWave waveform signals or data including vital sign data and/or point cloud data based that may be determined therefrom and as collected across a plurality of similarly situated users) in order to facilitate making predictions, classifications, and/or identifications for subsequent data (such as using the Al model on a specific person, such as person 110p1 or person 110p2, in order to determine or generate a classification or prediction for, or associated with, detecting a sleep classification, product classification, determine a trigger, event, or as otherwise described herein).

Machine learning model(s), such as the Al model described herein (e.g., the Al model of block 340) for some aspects, may be created and trained based upon example data inputs (e.g., "training data" comprising raw mmWave waveforms and/or processes mmWave waveforms, including vital sign data and point cloud data as described herein), which may be termed "features" and "labels" in order to make valid and reliable predictions for new inputs, such as testing level or production level data or inputs. In supervised machine learning, a machine learning program operating on a server, mobile device, or otherwise processor(s), may be provided with example inputs (e.g., "features") and their associated, or observed, outputs (e.g., "labels") in order for the machine learning program or algorithm to determine or discover rules, relationships, patterns, or otherwise machine learning "models" that map such inputs (e.g., "features") to the outputs (e.g., labels), for example, by determining and/or assigning weights or other metrics to the model across its various feature categories. Such rules, relationships, or otherwise models may then be provided subsequent inputs in order for the model, executing on a server (e.g., of a cloud platform), mobile device, or otherwise processor(s) (e.g., of mmWave mapping device 102), to predict, based on the discovered rules, relationships, or model, an expected output.

In unsupervised machine learning, the server, mobile device, or otherwise processor(s), may be required to find its own structure in unlabeled example inputs, where, for example multiple training iterations are executed by the server, mobile device, or otherwise processor(s) to train multiple generations of models until a satisfactory model, e.g., a model that provides sufficient prediction accuracy when given test level or production level data or inputs, is generated.

Supervised learning and/or unsupervised machine learning may also comprise retraining, relearning, or otherwise updating models with new, or different, information, which may include information received, ingested, generated, or otherwise used over time. The disclosures herein may use one or both of such supervised or unsupervised machine learning techniques.

For example, at block 340, in various aspects, an artificial intelligence (AI) model is trained with each of training point cloud data generated based on mmWave waveforms (e.g., as described for blocks 306, 308, and/or 310, and/or elsewhere herein) and vital sign data determined based on mmWave waveforms (e.g., as described for blocks 320-330 and/or elsewhere herein). In some aspects, the Al model (e.g., sleep classification model) is further trained with a second training dataset (e.g., environment dataset) as generated by a second sensor (e.g., sensors as described for blocks 342 and 344), where the Al model is configured to receive a new second data set generated by a second sensor (e.g., environmental signals or data such as room humidity and room temperature as input) as input and further configured to output a sleep phase classification further based on the new second data set.

The Al model is configured to receive point cloud data of a person (e.g., person 110p2, the child) and the one or more vital signs of the person (e.g., person 110p2) as input. As shown for block 340, the Al model is further configured to output a sleep phase classification corresponding to the human psychological state as defined by the point cloud data of the person and the one or more vital signs of the person, and, in some aspects, the environmental data as described.

In some aspects, the Al model is further trained with a plurality of user feedback responses defining sleep quality of respective users. In such aspects, the Al model is configured to receive one or more feedback responses of the person (e.g., person 110p2, the mother) as input and output the sleep quality classification further based on the one or more feedback responses of the person. In this way, the person (e.g., person 110p2) may provide user feedback of sleep quality as input to influence generation or modification sleep quality classification.

At block 350, the Al model (e.g., sleep classification model) is further configured to output a sleep quality classification. The sleep quality classification is based on the sleep phase classification determined at a first time and a second sleep phase classification determined, by the Al model, at a second time. In this way, Al model (e.g., at blocks 310 and 340) determines sleep phases over time to predict or classify sleep quality or other sleep metrics as described herein.

In some aspects, a set of computing instructions, when executed by the one or more processors (e.g., mmWave mapping device 102), may cause the one or more processors to generate a sleep deviation value based on the sleep phase classification determined at the first time and the second sleep phase classification determined at the second time. The sleep deviation value may be determined by a comparison of sleep quality over span of time (days, weeks) to provide the deviation of sleep quality for the given person.

For example, further at block350, the sleep phase classification and/or sleep quality classification, or other output of Al model, may comprise classifications or information that may be used as output, or a basis, to provide electronic feedback regarding the person's sleep quality, vitals, and/or posture, or otherwise as described herein. In some aspects, for example, output may comprise quality of sleep based on sleeping metrics, vitals, posture (e.g., the electronic feedback may indicate to the user that that user (e.g., person 110p2) has a restless sleep quality (e.g., at 20% optimal sleep) or good sleep quality (e.g., at 90% optimal sleep).

At block 352, a user may provide feedback or otherwise data to interact with mmWave mapping system 100 and/or mmWave mapping method 300. Such feedback or data may comprise a user input as to the user's perceived quality of sleep. Additionally, or alternatively, such feedback or data may comprise data such as body mass index (BMI) of persons in the room, age, and/or height or the like. Such feedback or data may be provided by the user via a display or GUI, e.g., as described for FIG. 4 herein. Such feedback or data may be used for the current iteration or for further or future iterations of mmWave mapping method 300, or otherwise as described herein for mmWave mapping system 100 and/or mmWave mapping method 300, where such data is used together with mmWave data and/or point cloud data to enhance or at least augment output, such as sleep phase classification(s) or otherwise as described herein.

In some aspects, a set of computing instructions, when executed by the one or more processors (e.g., of mmWave mapping device 102, mobile device 400, and/or of mmWave mapping system 100), may further cause the one or more processors to generate a user-specific sleep recommendation based on the sleep quality classification. For example, the user-specific sleep recommendation may comprise informing a person (e.g., person 110p2, the mother) to sleep on her back in order to experience more restful sleep. More generally, the mmWave mapping method 300 may use posture information and sleep quality predictions to derive learnings of good sleep and recommend a best posture for sleep as personalized feedback to user (e.g., person 110p2) to achieve a best sleep quality.

In some aspects, a set of computing instructions, when executed by the one or more processors (e.g., of mmWave mapping device 102, mobile device 400, and/or of mmWave mapping system 100), may cause the one or more processors to update, based on the sleep quality classification, a state of a device (e.g., mmWave mapping device 102) communicatively coupled to the one or more processors. For example, updating the state of the device (e.g., mmWave mapping device 102) may comprise activating or deactivating the device, changing a color or light device (e.g., via by updating LEDs of the device), and/or playing audio (e.g., relaxing music) to calm a user (e.g., person 110p2) if a low sleep quality is detected.

In other aspects, mmWave mapping method 300 may comprise measuring psychological state of person over time to provide feedback on changes of state. For example, in some aspects, a set of computing instructions, when executed by the one or more processors (e.g., of mmWave mapping device 102, mobile device 400, and/or of mmWave mapping system 100), further cause the one or more processors to generate a second point cloud (e.g., a second version or iteration of point cloud 206) that maps at least a portion of the physical space (e.g., physical space 104). The second point cloud may comprise second point cloud data defining the person or the object detected within the physical space (e.g., person 110p1, person 110p2, furniture 121, etc.) where the second point cloud is generated at a second time period based on second one or more mmWave waveforms of the mmWave sensor (e.g., mmWave sensor 202). The computing instructions, when executed by the one or more processors, may further case the one or more processors to determine, based on the second point cloud data, a second posture of the person within the portion of the physical space. The computing instructions, when executed by the one or more processors, may further cause the one or more processors to determine, based on the second one or more mmWave waveforms of the mmWave sensor, a second one or more vital signs of the person, and further determine the human psychological state as further defined by the second point cloud data and the second one or more vital signs of the person. The computing instructions, when executed by the one or more processors, may further cause the one or more processors to provide a second electronic feedback representing the human psychological state as defined by the second point cloud data at the second time period and the second one or more vital signs of the person. In some aspects, the computing instructions, when executed by the one or more processors, further cause the one or more processors to generate a state measurement value measuring a difference between a quality or quantity of the human psychological state between a first time period and the second time period.

In various aspects, mmWave mapping method 300 comprises providing an electronic feedback, for example as described for FIG. 4, representing the human psychological state as defined by the point cloud data and the one or more vital signs of the person. In some aspects, the electronic feedback comprises visual feedback. Additionally, or alternatively, the electronic feedback may comprise audio feedback.

FIG. 4 illustrates example electronic feedback of the mmWave mapping system of FIG. 1 and 2A, and mmWave mapping method of FIG. 3 wherein the electronic feedback comprises visual feedback as displayed on a graphic user interface (GUI), in accordance with various aspects disclosed herein. In the example of FIG. 4, GUI 402 is rendered or displayed on a screen of a mobile device (e.g., mobile device 400). Mobile device 400 may implement or execute an operating system (OS) or mobile platform such as APPLE's iOS and/or GOOGLE's Android operation system. Mobile device 400 may comprise one or more processors and/or one or more memories for storing, implementing, or executing computing instructions, e.g., comprising one or more mobile apps. For example, a set of mobile app instructions may be stored locally on a memory of a user mobile device (e.g., mobile device 400). In various aspects, the mobile app instructions of a mobile app of mobile device 400 may comprise computing app code or apps programmed in a programming language for the operating system (OS) of the underlying mobile device, which can include Objective-C and/or SWIFT for APPLE iOS based devices, Java for GOOGLE ANDROID devices, and the like.

The mobile app of mobile device 400 may comprise a portion of the application (app) of the mmWave mapping system and methods, as described herein for FIGs. 1, 2A, 2B, 2C, and 3. In particular, in various aspects, mobile device 400 is communicatively coupled via electronic communication 422 (e.g., wirelessly via BLUETOOTH OR WIFI (802.11) or wired via USB or otherwise) to the mmWave mapping device 102. Additionally, or alternatively, in some embodiments, mobile device 400 is connected via computer network 520 to server(s) 502 as described herein for FIG. 5. In various aspects, the mobile app comprises computing instructions, executable on one or more processors of mobile device 400, for receiving data and information, including vital sign data, point cloud data, and/or other data and/or output as described herein for FIGS. 1, 2A, 2B, 2C, and/or 3 and may render the data or representations thereof on GUI 402. The mobile app of mobile device 400 may be downloaded from app stores (e.g., APPLE APP STORE and/or GOOGLE PLAY) and installed on mobile device 400.

Additionally, or alternatively, GUI 402 may be implemented or rendered via a web interface, such as via a web browser application, e.g., Safari and/or GOOGLE Chrome app(s), or other such web browser or the like.

In some aspects, the computing instructions, as described herein for any of FIGS. 1, 2A, 2B, 2C, and/or 3 may be fully, or at least partially, implemented on or by the mobile app of device 400. Additionally, or alternatively, the computing instructions, as described herein for any of FIGS. 1, 2A, 2B, 2C, and/or 3 may be fully, or at least partially, implemented on or by the mmWave mapping device 102, and/or, more generally, mmWave mapping system 100, including server(s), such as server(s) of a cloud platform (e.g., MICROSOFT AZURE platform) as described herein. It is to be understood that the computing instructions, as described herein, may be located at and implemented on, and/or otherwise be a part of, any one or more of the mobile app of mobile device 400, mmWave mapping device 102, and/or more generally mmWave mapping system 100, such as server(s) of a cloud platform, where various implementations of the computing instructions across these systems and device are contemplated. The computing instructions may be configured to communicate across computer and/or wireless networks in order to transmit the data, including vital signs, point cloud data, mmWave signal data, or otherwise as described herein to implement any of the flow charts, algorithms, use or training of Al models, or otherwise as described herein. For example, in some aspects mobile device 400 is communicatively coupled via a base station and computer network. A base station may comprise a cellular base station, such as cell towers, communicating to mobile device 400 via wireless communications based on any one or more of various mobile phone standards, including NMT, GSM, CDMA, UMMTS, LTE, 5G, or the like. Additionally or alternatively, a base station may comprise routers, wireless switches, or other such wireless connection points communicating to the mobile device 400 based on any one or more of various wireless standards, including by non-limiting example, IEEE 802.11a/b/c/g (WIFI), the BLUETOOTH standard, or the like.

As shown in the example of FIG. 4, the mobile app is configured to display, on display screen 401, example electronic feedback of the mmWave mapping system of FIG. 1 and 2A, and mmWave mapping method of FIG. 3, wherein the electronic feedback comprises visual feedback as displayed on GUI 402. As shown in FIG. 4, graphical physical space 104g is rendered on GUI 402 depicting each of graphic 110p1g and graphic 110p2g corresponding to person 110p1 and person 110p2 within physical space 104, respectively.

In addition, as shown in FIG. 4, human psychological states 404 of the persons of represented by graphical physical space 104g are provided via text area 406. Human psychological state(s) may comprise one or more of: a sleep state, a restfulness state, a restlessness state, a stress state, a sadness state, or a happiness state. More generally, the human psychological state(s) are output by mmWave mapping system 100 that express the ability of mmWave mapping system 100 to measure and feedback different sleep states and/or stages. The human psychological state could be sleep state (e.g., defining various states, stress, sadness, happiness).

In additional, aspects, the set of computing instructions, when executed by the one or more processors (e.g., processors of mobile device 400, mmWave mapping device 102, or otherwise mmWave mapping system 100), further cause the one or more processors to generate a user-specific score (e.g., a sleep score or sleep quality score to of the person) defining a quality or quantity of the human psychological state as defined by one or more of the point cloud data or the one or more vital signs of the person. In some aspects, the user-specific score is rendered on a GUI (e.g., GUI 402), where electronic feedback comprises visual feedback as displayed by GUI 400.

For example, in FIG. 4, mmWave mapping system 100 has detected, and as shown in text area 406, based on mmWave waveforms, vital signs, and/or point cloud data as described herein, that person 100p1 (e.g., the mother) is currently in a restlessness state (with a user-specific score of 55% restlessness) and also in a stress state (with a user specific score of 35% stress).

As a further example, mmWave mapping system 100 has detected, and as shown in text area 406, based on mmWave waveforms, vital signs, and/or point cloud data as described herein, that person 100p2 (e.g., the child) is currently in a restfulness state (with a user-specific score of 90% restlessness) and also in a sleep state (with a user specific score of 95% sleep).

As illustrated herein, in some aspects, electronic feedback may comprise a visual feedback configured for display on a GUI (e.g., GUI 402). Additionally, or alternatively, an electronic feedback may comprise an audio feedback. The audio feedback may be configured for audio output on an audio device (e.g., such as an ALEXA or GOOGLE device, or a LUMI device) comprising one or more speakers.

In some aspects, the set of computing instructions, when executed by the one or more processors (e.g., processors of mobile device 400, mmWave mapping device 102, or otherwise mmWave mapping system 100), further cause the one or more processors to receive an input specifying control of the electronic feedback, where the input causes one or more of a starting or stopping of the electronic feedback, a frequency of the electronic feedback, or a pattern of providing or displaying the electronic feedback. For example, in various aspects, mmWave mapping system 100 may be controlled to start or stop response output, increase or decrease response output, specify a response frequency, and/or specify a response pattern. In some aspects, update button 410 may cause mmWave mapping system 100 to generate a response or update or the graphical physical space 104g and/or text area based on additional mmWave processing and detection as described herein. In other aspects, such update is automatic and does not require interaction with a button.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A millimeter wave (mmWave) mapping system configured to generate one or more point clouds and to determine one or more vital signs for defining a human psychological state, the mmWave mapping system comprising:
an mmWave sensor (202) configured to transmit and receive one or more mmWave waveforms within a physical space;
one or more processors commutatively coupled to the mmWave sensor, wherein a digital signal processor of the one or more processors is configured to analyze the one or more mmWave waveforms; and
an application (app) (508) comprising a set of computing instructions, the set of computing instructions configured for execution by the one or more processors, and the set of computing instructions, when executed by the one or more processors, cause the one or more processors to:
generate, based on one or more mmWave waveforms of the mmWave sensor, a time-resolved (4D) point cloud that maps at least a portion of the physical space, the 4D point cloud comprising a space dimension and a time value, wherein the point cloud comprises point cloud data defining a person detected within the physical space,
determine, based on the point cloud data defining the person, a posture of the person within the portion of the physical space, wherein determining the posture comprises segmenting the point cloud by Al to identify a person cluster,
determine, based on the one or more mmWave waveforms of the mmWave sensor, one or more vital signs of the person, wherein determining the vital signs comprises selecting point-cloud elements corresponding to the person's torso and computing phase and amplitude changes to determine a chest displacement of the person,
determine a human psychological state of the person as defined by the point cloud data and the one or more vital signs of the person, and
provide an electronic feedback representing the human psychological state as defined by the point cloud data and the one or more vital signs of the person,
wherein the mmWave mapping system further comprises an artificial intelligence (AI) model, wherein the Al model is trained with each of training point cloud data generated based on mmWave waveforms and vital sign data determined based on mmWave waveforms, and wherein the Al model is configured to receive the point cloud data of the person and the one or more vital signs of the person as input and is further configured to output a sleep phase classification corresponding to the human psychological state as defined by the point cloud data of the person and the one or more vital signs of the person, and wherein the mmWave mapping system does not comprise any optical imaging sensor including a camera, and does not comprise any microphone or audio sensor.

2. The mmWave mapping system of any one of claims 1, wherein the human psychological state comprises one or more of: a sleep state, a restfulness state, a restlessness state, a stress state, a sadness state, or a happiness state.

3. The mmWave mapping system of any one of claims 1-2, wherein the set of computing instructions, when executed by the one or more processors, further cause the one or more processors to: generate a user-specific score defining a quality or quantity of the human psychological state as defined by one or more of: the point cloud data or the one or more vital signs of the person.

4. The mmWave mapping system of claim 3, wherein the user-specific score is rendered on a graphical user interface (GUI), and wherein the electronic feedback comprises visual feedback as displayed by the GUI.

5. The mmWave mapping system of any one of claims 1-3, wherein the electronic feedback comprises a visual feedback, and wherein the visual feedback is configured for display on a graphic user interface (GUI).

6. The mmWave mapping system of any one of claims 1-5, wherein the electronic feedback comprises an audio feedback, and wherein the audio feedback is configured for audio output on an audio device comprising one or more speakers.

7. The mmWave mapping system of any one of claims 1-6, wherein the set of computing instructions, when executed by the one or more processors, further cause the one or more processors to: generate a second point cloud that maps at least a portion of the physical space, wherein the second point cloud comprises second point cloud data defining the person or the object detected within the physical space, and wherein the second point cloud is generated at a second time period based on second one or more mmWave waveforms of the mmWave sensor, determine, based on the second point cloud data, a second posture of the person within the portion of the physical space, determine, based on the second one or more mmWave waveforms of the mmWave sensor, a second one or more vital signs of the person, determine the human psychological state as further defined by the second point cloud data and the second one or more vital signs of the person, and provide a second electronic feedback representing the human psychological state as defined by the second point cloud data at the second time period and the second one or more vital signs of the person.

8. The mmWave mapping system of claim 7, wherein the set of computing instructions, when executed by the one or more processors, further cause the one or more processors to: generate a state measurement value measuring a difference between a quality or quantity of the human psychological state between a first time period and the second time period.

9. The mmWave mapping system of any one of claims 1-8, wherein the set of computing instructions, when executed by the one or more processors, further cause the one or more processors to: determine a chest displacement of the person based on the vital signs of the person.

10. The mmWave mapping system of claim 9, wherein the set of computing instructions, when executed by the one or more processors, further cause the one or more processors to: determine a breathing rate or a heart rate based on the chest displacement.

11. The mmWave mapping system of any one of claims 1-10, wherein the set of computing instructions, when executed by the one or more processors, further cause the one or more processors to: determine at least one of: a type of the posture of the person, a volumetric size of the person, an area of the person, or a representation of a current posture of the person.

13. The mmWave mapping system of any one of claims 1-12, wherein the set of computing instructions, when executed by the one or more processors, further cause the one or more processors to: receive an input specifying control of the electronic feedback, where the input causes one or more of: a starting or stopping of the electronic feedback, a frequency of the electronic feedback, or a pattern of providing or displaying the electronic feedback.

14. The mmWave mapping system of any one of claims 1-13, wherein at least one of the mmWave sensor or the one or more processors is positioned within or is in a proximity to a furniture within the physical space, and preferably the furniture is a baby crib.

## Patentansprüche

1. Millimeterwellen-Mapping-System (mmWave-Mapping-System), das konfiguriert ist, um eine oder mehrere Punktwolken zu erzeugen und um ein oder mehrere Vitalzeichen zum Bestimmen eines menschlichen psychologischen Zustands zu ermitteln, das mmWave-Mapping-System umfassend:
einen mmWave-Sensor (202), der konfiguriert ist, um eine oder mehrere mmWave-Wellenformen innerhalb eines physikalischen Raums zu übertragen und zu empfangen;
einen oder mehrere Prozessoren, die mit dem mmWave-Sensor kommunikativ gekoppelt sind, wobei ein digitaler Signalprozessor des einen oder der mehreren Prozessoren konfiguriert ist, um die eine oder die mehreren mmWave-Wellenformen zu analysieren; und
eine Anwendung (App) (508), umfassend einen Satz von Rechenanweisungen, wobei der Satz von Rechenanweisungen für eine Ausführung durch den einen oder die mehreren Prozessoren konfiguriert ist, und der Satz von Rechenanweisungen, wenn er durch den einen oder die mehreren Prozessoren ausgeführt wird, den einen oder die mehreren Prozessoren veranlasst zum:
Erzeugen, basierend auf einer oder mehreren mmWave-Wellenformen des mmWave-Sensors, einer zeitaufgelösten Punktwolke (4D-Punktwolke), die wenigstens einen Teil des physikalischen Raums mappt, die 4D-Punktwolke umfassend eine Raumabmessung und einen Zeitwert, wobei die Punktwolke Punktwolkendaten umfasst, die eine Person bestimmen, die innerhalb des physikalischen Raums detektiert wird,
Ermitteln, basierend auf den Punktwolkendaten, die die Person bestimmen, einer Körperhaltung der Person innerhalb des Teils des physikalischen Raums, wobei das Ermitteln der Körperhaltung ein Segmentieren der Punktwolke durch KI umfasst, um einen Personencluster zu identifizieren,
Ermitteln, basierend auf der einen oder den mehreren mmWave-Wellenformen des mmWave-Sensors, eines oder mehrerer Vitalzeichen der Person, wobei das Ermitteln der Vitalzeichen ein Auswählen von Punktwolkenelementen, die dem Torso der Person entsprechen, und ein Berechnen von Phasen- und Amplitudenänderungen umfasst, um eine Brustverschiebung der Person zu ermitteln,
Ermitteln eines menschlichen psychologischen Zustands der Person, wie durch die Punktwolkendaten und das eine oder die mehreren Vitalzeichen der Person bestimmt, und
Bereitstellen einer elektronischen Rückkopplung, die den menschlichen psychologischen Zustand, wie durch die Punktwolkendaten und das eine oder die mehreren Vitalzeichen der Person bestimmt, darstellt,
wobei das mmWave-Mapping-System ferner ein Künstliche-Intelligenz-Modell (KI-Modell) umfasst, wobei das KI-Modell mit jedem von Trainingspunktwolkendaten, die basierend auf mmWave-Wellenformen erzeugt werden, und Vitalzeichendaten, die basierend auf mmWave-Wellenformen ermittelt werden, trainiert wird und wobei das KI-Modell konfiguriert ist, um die Punktwolkendaten der Person und das eine oder die mehreren Vitalzeichen der Person als Eingabe zu empfangen, und ferner konfiguriert ist, um eine Schlafphasenklassifikation, die dem menschlichen psychologischen Zustand entspricht, wie durch die Punktwolkendaten der Person und dem einen oder den mehreren Vitalzeichen der Person bestimmt, auszugeben, und wobei das mmWave-Mapping-System keinen optischen Bildgebungssensor umfasst, der eine Kamera einschließt, und kein Mikrofon oder keinen Audiosensor umfasst.

2. mmWave-Mapping-System nach einem der Ansprüche 1, wobei der menschliche psychologische Zustand einen oder mehrere umfasst von: einem Schlafzustand, einem Ruhezustand, einem Unruhezustand, einem Stresszustand, einem Traurigkeitszustand oder einem Glückszustand.

3. mmWave-Mapping-System nach einem der Ansprüche 1 bis 2, wobei der Satz von Rechenanweisungen, wenn er durch den einen oder die mehreren Prozessoren ausgeführt wird, ferner den einen oder die mehreren Prozessoren veranlasst: eine benutzerspezifische Wertung zu erzeugen, die eine Qualität oder eine Quantität des menschlichen psychologischen Zustands bestimmt, wie durch eines oder mehrere bestimmt von: den Punktwolkendaten oder dem einen oder den mehreren Vitalzeichen der Person.

4. mmWave-Mapping-System nach Anspruch 3, wobei die benutzerspezifische Wertung auf einer grafischen Benutzeroberfläche (GUI) gerendert wird und wobei die elektronische Rückkopplung eine visuelle Rückkopplung, wie durch die GUI angezeigt, umfasst.

5. mmWave-Mapping-System nach einem der Ansprüche 1 bis 3, wobei die elektronische Rückkopplung eine visuelle Rückkopplung umfasst und wobei die visuelle Rückkopplung für eine Anzeige auf einer grafischen Benutzeroberfläche (GUI) konfiguriert ist.

6. mmWave-Mapping-System nach einem der Ansprüche 1 bis 5, wobei die elektronische Rückkopplung eine Audiorückkopplung umfasst und wobei die Audiorückkopplung für eine Audioausgabe auf einer Audiovorrichtung konfiguriert ist, umfassend einen oder mehrere Lautsprecher.

7. mmWave-Mapping-System nach einem der Ansprüche 1 bis 6, wobei der Satz von Rechenanweisungen, wenn er durch den einen oder die mehreren Prozessoren ausgeführt wird, den einen oder die mehreren Prozessoren ferner veranlasst: eine zweite Punktwolke zu erzeugen, die wenigstens einen Teil des physikalischen Raums mappt, wobei die zweite Punktwolke zweite Punktwolkendaten umfasst, die die Person oder das Objekt bestimmen, die/das innerhalb des physikalischen Raums detektiert wird, und wobei die zweite Punktwolke in einer zweiten Zeitspanne basierend auf einer zweiten einen oder zweiten mehreren mmWave-Wellenformen des mmWave-Sensors erzeugt wird, basierend auf den zweiten Punktwolkendaten eine zweite Körperhaltung der Person innerhalb des Teils des physikalischen Raums zu ermitteln, basierend auf der zweiten einen oder den zweiten mehreren mmWave-Wellenformen des mmWave-Sensors ein zweites eines oder zweite mehrere Vitalzeichen der Person zu ermitteln, den menschlichen psychologischen Zustand, wie durch die zweiten Punktwolkendaten und das zweite eine oder die zweiten mehreren Vitalzeichen der Person ferner bestimmt, zu ermitteln und eine zweite elektronische Rückkopplung bereitzustellen, die den menschlichen psychologischen Zustand, wie durch die zweiten Punktwolkendaten in der zweiten Zeitspanne und das zweite eine oder die zweiten mehreren Vitalzeichen der Person bestimmt, darstellt.

8. mmWave-Mapping-System nach Anspruch 7, wobei der Satz von Rechenanweisungen, wenn er durch den einen oder die mehreren Prozessoren ausgeführt wird, den einen oder die mehreren Prozessoren ferner veranlasst: einen Zustandsmesswert zu erzeugen, der eine Differenz zwischen einer Qualität oder einer Quantität des menschlichen psychologischen Zustands zwischen einer ersten Zeitspanne und der zweiten Zeitspanne misst.

9. mmWave-Mapping-System nach einem der Ansprüche 1 bis 8, wobei der Satz von Rechenanweisungen, wenn er durch den einen oder die mehreren Prozessoren ausgeführt wird, den einen oder die mehreren Prozessoren ferner veranlasst: eine Brustverschiebung der Person basierend auf den Vitalzeichen der Person zu ermitteln.

10. mmWave-Mapping-System nach Anspruch 9, wobei der Satz von Rechenanweisungen, wenn er durch den einen oder die mehreren Prozessoren ausgeführt wird, den einen oder die mehreren Prozessoren ferner veranlasst: eine Atemfrequenz oder eine Herzfrequenz basierend auf der Brustverschiebung zu ermitteln.

11. mmWave-Mapping-System nach einem der Ansprüche 1 bis 10, wobei der Satz von Rechenanweisungen, wenn er durch den einen oder die mehreren Prozessoren ausgeführt wird, den einen oder die mehreren Prozessoren ferner veranlasst: wenigstens eines zu ermitteln von: einer Art der Körperhaltung der Person, einer volumetrischen Größe der Person, einer Fläche der Person oder einer Darstellung einer aktuellen Körperhaltung der Person.

13. mmWave-Mapping-System nach einem der Ansprüche 1 bis 12, wobei der Satz von Rechenanweisungen, wenn er durch den einen oder die mehreren Prozessoren ausgeführt wird, den einen oder die mehreren Prozessoren ferner veranlasst: eine Eingabe zu empfangen, die eine Steuerung der elektronischen Rückkopplung spezifiziert, wobei die Eingabe eines oder mehrere veranlasst von: einem Starten oder einem Stoppen der elektronischen Rückkopplung, einer Schwingungszahl der elektronischen Rückkopplung oder einem Muster des Bereitstellens oder des Anzeigens der elektronischen Rückkopplung.

14. mmWave-Mapping-System nach einem der Ansprüche 1 bis 13, wobei wenigstens einer des mmWave-Sensors oder des einen oder der mehreren Prozessoren innerhalb eines Möbelstücks innerhalb des physikalischen Raums angeordnet ist oder in der Nähe davon ist und das Möbelstück vorzugsweise ein Babybett ist.

## Revendications

1. Système de cartographie à ondes millimétriques (mmWave) configuré pour générer un ou plusieurs nuages de points et pour déterminer un ou plusieurs signes vitaux permettant de définir un état psychologique humain, le système de cartographie mmWave comprenant :
un capteur mmWave (202) configuré pour transmettre et recevoir une ou plusieurs formes d'onde mmWave au sein d'un espace physique ;
un ou plusieurs processeurs couplés en communication au capteur mmWave, dans lequel un processeur de signal numérique du ou des processeurs est configuré pour analyser la ou les formes d'ondes mmWave ; et
une application (app) (508) comprenant un ensemble d'instructions de calcul, l'ensemble d'instructions de calcul étant configuré pour une exécution par le ou les processeurs, et l'ensemble d'instructions de calcul, lorsqu'il est exécuté par le ou les processeurs, amène le ou les processeurs à :
générer, sur la base d'une ou plusieurs formes d'ondes mmWave du capteur mmWave, un nuage de points résolu dans le temps (4D) qui cartographie au moins une partie de l'espace physique, le nuage de points 4D comprenant une dimension spatiale et une valeur temporelle, dans lequel le nuage de points comprend des données de nuage de points définissant une personne détectée au sein de l'espace physique,
déterminer, sur la base des données de nuage de points définissant la personne, une posture de la personne au sein de la partie de l'espace physique, dans lequel la détermination de la posture comprend la segmentation du nuage de points par IA pour identifier un groupe de personnes,
déterminer, sur la base de la ou des formes d'ondes mmWave du capteur mmWave, un ou plusieurs signes vitaux de la personne, dans lequel la détermination des signes vitaux comprend la sélection d'éléments de nuage de points correspondant au torse de la personne et le calcul de changements de phase et d'amplitude pour déterminer un déplacement thoracique de la personne,
déterminer un état psychologique humain de la personne tel que défini par les données de nuage de points et le ou les signes vitaux de la personne, et
fournir un retour électronique représentant l'état psychologique humain tel que défini par les données de nuage de points et le ou les signes vitaux de la personne,
dans lequel le système de cartographie mmWave comprend en outre un modèle d'intelligence artificielle (IA), dans lequel le modèle IA est entraîné avec chacune des données de nuage de points d'entraînement générées sur la base de formes d'ondes mmWave et de données de signes vitaux déterminées sur la base de formes d'onde mmWave, et dans lequel le modèle IA est configuré pour recevoir les données de nuage de points de la personne et le ou les signes vitaux de la personne en tant qu'entrée et est en outre configuré pour délivrer une classification de phase de sommeil correspondant à l'état psychologique humain tel que défini par les données de nuage de points de la personne et le ou les signes vitaux de la personne, et dans lequel le système de cartographie mmWave ne comprend pas de capteur d'imagerie optique comportant une caméra, et ne comprend pas de microphone ou de capteur audio.

2. Système de cartographie mmWave selon l'une quelconque des revendications 1, dans lequel l'état psychologique humain comprend l'un ou plusieurs parmi : un état de sommeil, un état de repos, un état d'agitation, un état de stress, un état de tristesse, ou un état de bonheur.

3. Système de cartographie mmWave selon l'une quelconque des revendications 1 à 2, dans lequel l'ensemble d'instructions de calcul, lorsqu'il est exécuté par le ou les processeurs, amène en outre le ou les processeurs à : générer un score spécifique à l'utilisateur définissant une qualité ou quantité de l'état psychologique humain tel que défini par l'un ou plusieurs parmi : les données de nuage de points ou le ou les signes vitaux de la personne.

4. Système de cartographie mmWave selon la revendication 3, dans lequel le score spécifique à l'utilisateur est rendu sur une interface utilisateur graphique (GUI), et dans lequel le retour électronique comprend un retour visuel tel qu'affiché par la GUI.

5. Système de cartographie mmWave selon l'une quelconque des revendications 1 à 3, dans lequel le retour électronique comprend un retour visuel, et dans lequel le retour visuel est configuré pour un affichage sur une interface utilisateur graphique (GUI).

6. Système de cartographie mmWave selon l'une quelconque des revendications 1 à 5, dans lequel le retour électronique comprend un retour audio, et dans lequel le retour audio est configuré pour une sortie audio sur un dispositif audio comprenant un ou plusieurs haut-parleurs.

7. Système de cartographie mmWave selon l'une quelconque des revendications 1 à 6, dans lequel l'ensemble d'instructions de calcul, lorsqu'il est exécuté par le ou les processeurs, amène en outre le ou les processeurs à : générer un second nuage de points qui cartographie au moins une portion de l'espace physique, dans lequel le second nuage de points comprend des secondes données de nuage de points définissant la personne ou l'objet détecté au sein de l'espace physique, et dans lequel le second nuage de points est généré à un second laps de temps sur la base d'une ou de plusieurs secondes formes d'ondes mmWave du capteur mmWave, déterminer, sur la base des secondes données de nuage de points, une seconde posture de la personne au sein de la partie de l'espace physique, déterminer, sur la base de la ou des secondes formes d'ondes mmWave du capteur mmWave, un ou plusieurs seconds signes vitaux de la personne, déterminer l'état psychologique humain tel que défini en outre par les secondes données de nuage de points et le ou les seconds signes vitaux de la personne, et fournir un second retour électronique représentant l'état psychologique humain tel que défini par les secondes données de nuage de points au second laps de temps et le second ou les seconds signes vitaux de la personne.

8. Système de cartographie mmWave selon la revendication 7, dans lequel l'ensemble d'instructions de calcul, lorsqu'il est exécuté par le ou les processeurs, amène en outre le ou les processeurs à : générer une valeur de mesure d'état mesurant une différence entre une qualité ou quantité de l'état psychologique humain entre un premier laps de temps et le second laps de temps.

9. Système de cartographie mmWave selon l'une quelconque des revendications 1 à 8, dans lequel l'ensemble d'instructions de calcul, lorsqu'il est exécuté par le ou les processeurs, amène en outre le ou les processeurs à : déterminer un déplacement thoracique de la personne sur la base des signes vitaux de la personne.

10. Système de cartographie mmWave selon la revendication 9, dans lequel l'ensemble d'instructions de calcul, lorsqu'il est exécuté par le ou les processeurs, amène en outre le ou les processeurs à : déterminer une fréquence respiratoire ou une fréquence cardiaque sur la base du déplacement thoracique.

11. Système de cartographie mmWave selon l'une quelconque des revendications 1 à 10, dans lequel l'ensemble d'instructions de calcul, lorsqu'il est exécuté par le ou les processeurs, amène en outre le ou les processeurs à : déterminer au moins l'un parmi : un type de la posture de la personne, une taille volumétrique de la personne, une surface de la personne, ou une représentation d'une posture actuelle de la personne.

13. Système de cartographie mmWave selon l'une quelconque des revendications 1 à 12, dans lequel l'ensemble d'instructions de calcul, lorsqu'il est exécuté par le ou les processeurs, amène en outre le ou les processeurs à : recevoir une entrée de spécification de commande du retour électronique, où l'entrée amène l'un ou plusieurs parmi : un démarrage ou un arrêt du retour électronique, une fréquence du retour électronique, ou un modèle de fourniture ou d'affichage du retour électronique.

14. Système de cartographie mmWave selon l'une quelconque des revendications 1 à 13, dans lequel au moins l'un du capteur mmWave ou du ou des processeurs est positionné au sein ou à proximité d'un meuble au sein de l'espace physique, et de préférence le meuble est un lit pour bébé.
